# FASCICULE DE BREVET EUROPEEN

(11) **EP 1 027 921 B1**
(45) Date de publication et mention de la délivrance du brevet: **12.01.2005**
(21) Numéro de dépôt: 00400222.6
(22) Date de dépôt: 28.01.2000
(51) Int. Cl.: B01F 17/00

(54) **Emulsion stable, son procédé de préparation à cet effet et agent émulsionnant**
Stabile Emulsion,Verfahren zur Herstellung und Emulgiermittel
Stable emulsion, method for producing it and the emulsifier

(30) Priorité: 10.02.1999 FR 9901553
(43) Date de publication de la demande: 16.08.2000
(73) Titulaire: Agro Industrie Recherches et Développement ( A.R.D.) Société Anonyme, 51110 Pomacle (FR)
(72) Inventeur: Bertho, Jean-Noel, 51110 Pomacle (FR); Mathaly, Philippe, 51100 Reims (FR); Baynast, Régis de, 78000 Versailles (FR); Dubois, Véronique, 58200 Cosne sur Loire (FR)
(74) Mandataire: Eidelsberg, Victor Albert

(56) Documents cités:
- EP-A- 0 628 305
- EP-A- 0 629 396
- EP-A- 0 895 805

## Description

La présente invention concerne des émulsions parfaitement stables dans le temps préparées à partir d'un émulsionnant comprenant au moins un polyglycosides et au moins un alcool gras, un procédé pour rendre stables les émulsions et un agent émulsionnant.

Les émulsions sont largement produites et utilisées dans l'industrie soit comme matériaux à consommer, soit à appliquer sur des surfaces en tant que vecteurs d'agents non solubles dans l'eau. On retrouve des émulsions en cosmétique (laits, 5 crèmes, pommades), en cuisine (sauces, crèmes), en galénique (pommades, crèmes), en peinture (peinture sans odeur), dans l'industrie routière (bitume en émulsion), en agrochimie (produits phytosanitaires), en détergence, dans le laminage, la sidérurgie et dans la fabrication de dépôts divers (imprimerie, adhésifs ...).

En cosmétique et en pharmacie, pour la conception de produits d'hygiène ou de soins, les émulsions constituent un moyen efficace pour obtenir la combinaison harmonieuse 5 d'ingrédients de nature et de propriétés différentes en une présentation homogène et facile d'emploi. Les émulsionnants les plus utilisés à ce jour sont les sulfates et les sulfonates d'alkyle, les alcools, les acides, les esters gras éthoxylés, les esters gras de sorbitan...

De nombreux composés phytosanitaires sont insolubles dans l'eau et, étant préalablement solubilisés dans un solvant organique, ils pourront être émulsionnés dans l'eau au moment de l'application ou de la formulation par un choix approprié d'émulsionnants.

C'est dans les présentations liquides de produits phytosanitaires que l'on retrouve le plus d'agents émulsionnants. Le concentré émulsionnable qui est la forme la plus courante et encore la plus usitée sur le marché actuel contient classiquement 250 g/l de pesticides par exemple et 50 g/l d'émulsionnants. La mise en oeuvre correspond à la formation d'une émulsion fine dont la stabilité doit être assurée pendant plusieurs heures indépendamment de la température ou de la dureté de l'eau. Les suspensions concentrées sont de développement plus récent et correspondent à la génération des formulations permettant d'appliquer de très faibles doses à l'hectare. C'est le cas par exemple des émulsions concentrées qui contiennent respectivement de 400 à 600 g/l de pesticides et de 50 à 100 g/l d'émulsionnants. Contrairement aux deux présentations précédentes, les microémulsions sont des systèmes thermodynamiquement stables, donc très intéressantes sur le plan du stockage des produits.

Les émulsionnants utilisés pour le domaine phytosanitaire sont essentiellement, en anioniques, le dodécylbenzènesulfonate de calcium, les alkylarylsulfonates d'amines, les phosphates esters d'alcool gras éthoxylés ou d'alkylphénols éthoxylés. En non ioniques, les plus utilisés sont les alkylphénols éthoxylés, les alcools et acides gras éthoxylés.

La préparation des émulsions au moyen des compositions contenant des polyglycosides et au moins un alcool gras est désormais bien connue (WO 92/06778, WO 96/37285, WO 95/13863, WO 98/47610, WO 97/18033, DE 196 07 977).

Ces tensioactifs, émulsionnants, peuvent se présenter sous la forme de compositions à base d'alcools gras et de polyglycosides qui présentent l'avantage d'être "auto-émulsionnable". On entend par "auto-émulsionnable" une composition qui permet d'obtenir une émulsion par simple mélange sous agitation modérée avec une phase aqueuse.

Cependant, il a été constaté, notamment dans EP 0628305, que l'utilisation de telles compositions émulsionnantes, ne permettait pas toujours d'obtenir des émulsions suffisamment stables dans le temps.

La demanderesse a également constaté que l'utilisation de compositions émulsionnantes de l'art antérieur ne permettait pas d'obtenir toujours des émulsions stables en présence d'huiles de silicones. Ces huiles sont cependant intéressantes, puisqu'elles permettent d'obtenir des émulsions au toucher agréable, présentant une bonne pénétration et généralement résistantes à l'eau.

Il est alors proposé, dans la présente invention, l'utilisation d'agents émulsionnants à base de polyglycosides et d'alcools gras, permettant l'obtention d'émulsions stables (c'est-à-dire ne présentant pas de déphasage après trois mois de vieillissement à 45° C) même en utilisant moins de 5 % en poids par rapport au poids total de l'émulsion de tels agents émulsionnants et même en présence d'huiles silicones.

La bonne stabilité des émulsions a pu être obtenue grâce à l'utilisation d'agent émulsionnants à base d'alcool gras et de polyglycosides contenant des polypentosides, choisis parmi les polyarabinosides et les polyxylosides, en une proportion bien déterminée.

En outre, la demanderesse a constaté que les émulsions suivant l'invention, préparées à partir d'agent émulsionnant à base d'alcool gras et de polyglycosides, présentaient un bon pouvoir suspensif, c'est à dire qu'elles permettent de maintenir en suspension des particules solides et ceci indépendamment de la viscosité de l'émulsion

Par ailleurs, les agents émulsionnants de l'invention, constituées de polyglycosides et d'alcools gras sont d'origine naturelle. Ils permettent la préparation d'émulsions stables totalement d'origine végétale. Ils ne présentent pas les inconvénients des émulsionnants à base de composés polyoxyéthylénés qui sont susceptibles de contenir des résidus de dioxanne. Ils ne sont pas irritants, ils ne sont pas toxiques et ils sont biodégradables.

En outre, les agents émulsionnants selon l'invention peuvent être utilisés comme compositions auto-émulsionnables.

La présente invention a donc pour objet des émulsions comprenant, en poids :
- de 4,5 à 99,5% d'eau
- de 0 à 95% d'huile
- de 0 à 50% de substance active
- un agent émulsionnant en complément à 100%,
l'agent émulsionnant comprend de 30 à 65% en poids d'au moins un alcool gras de formule ROH, où R est un radical aliphatique, saturé ou insaturé ayant de 1 à 4 insaturations éthyléniques, linéaire ou ramifié, ayant de 12 à 22 atomes de carbone, le reste étant, sauf des impuretés, constitué :
a)- soit, d' un mélange de polyglycosides comprenant des polypentosides de formule (I) :

   R¹O(P)ₙ₁ (I)

   dans laquelle R¹ est un radical aliphatique, saturé ou insaturé ayant de 1 à 4 insaturations éthyléniques, linéaire ou ramifié, ayant de 12 à 22 atomes de carbone, P est le reste d'un pentose choisi parmi l'arabinose et le xylose, n1 est compris entre 1 et 5 ; et des polyhexosides de formule (II) :

   R²O(H)ₙ₂ (II)

   dans laquelle R² est un radical aliphatique, saturé ou insaturé ayant de 1 à 4 insaturations éthyléniques, linéaire ou ramifié, ayant de 12 à 22 atomes de carbone, H est le reste d'un hexose, n2 est compris entre 1 et 5 ;
b)- soit d'un mélange de polyglycosides de formule (III):

   R³O(G₁)ₐ(G₂)_{b}(G₃)_{c}(G₄)_{d}(G₅)ₑ (III)

   dans laquelle R³ est un radical aliphatique, saturé ou insaturé ayant de 1 à 4 insaturations éthyléniques, linéaire ou ramifié, ayant de 12 à 22 atomes de carbone, G₁, G₂, G₃, G₄, G₅ sont indépendamment les uns des autres, des restes d'un ose choisi parmi les hexoses et les pentoses, ces derniers étant choisis parmi l'arabinose et le xylose ; a, b, c, d et e étant égaux à 0 ou à 1, la somme de a, b, c, d et e étant au moins égale à 1
c)- soit d'un mélange de a et b
caractérisée par les deux caractéristiques 1 et 2 suivantes :
1)lorsque l'agent émulsionnant comprend des polyglycosides comprenant des polypentosides de formule (I) et des polyhexosides de formule (II), les polypentosides de formule (I) représentent 66 à 100% en poids de l'ensemble des polyglycosides, et les polyhexosides de formule (II) 0 à 34% en poids de l'ensemble des polyglycosides, lorsque l'agent émulsionnant comprend des polyglycosides de formule (III), les pentoses représentent alors 66 à 100% en poids par rapport à l'ensemble des oses G₁, G₂, G₃, G₄ et G₅ et les hexoses de 0 à 34% en poids par rapport à l'ensemble des oses G₁, G₂, G₃, G₄ et G₅ et
2-a) ou bien l'agent émulsionnant représente au moins 2,5 % du poids total de l'émulsion. La phase huile de l'émulsion peut être alors constituée par une huile choisie parmi :
   - les huiles d'origine végétale, telles que l'huile d'amandes douces, l'huile de coprah, l'huile de ricin, l'huile de jojoba, l'huile d'olive, l'huile de colza, l'huile d'arachide, l'huile de noisette, l'huile de palme, le beurre de karité, l'huile de noyau d'abricot, l'huile de calophyllum, l'huile de carthame, l'huile d'avocat, l'huile de noix, l'huile de pépins de cassis, l'huile de germes de blé, l'huile de tournesol, l'huile de germes de maïs, l'huile de soja, l'huile de coton, l'huile de luzerne, l'huile d'orge, l'huile de pépins de raisin, l'huile de pavot, l'huile de potimarron, l'huile de sézame, l'huile de seigle, l'huile d'onagre, l'huile de passiflore, des dérivés de ces huiles comme les huiles hydrogénées,
   - les huiles d'origine animale (suif, huiles de poisson...),
   - les huiles minérales, telles que l'huile de paraffine, l'huile de vaseline et les huiles minérales notamment issues de coupes pétrolières.
   - les huiles synthétiques, comme les poly-α-oléfines,
   - les dérivés de la lanoline,
   - les alcanediols possédant de 2 à 10 atomes de carbone comme le 1,2-propanediol, le 1,3-butanediol,
   - les alcools de formule R⁴-OH où R⁴ est un radical aliphatique, saturé ou insaturé ayant de 1 à 4 insaturations éthyléniques, linéaire ou ramifié, ayant de 12 à 22 atomes de carbone, tels que l'alcool myristique, l'alcool cétylique, l'alcool stéarylique, l'alcool oléique,
   - les polyéthylène glycols ou polypropylène glycols,
   - les esters gras de formule R⁵-0-CO- R⁶ où R⁵ et R⁶ sont indépendamment l'un de l'autre des radicaux aliphatiques, saturés ou insaturés ayant de 1 à 4 insaturations éthyléniques, linéaires ou ramifiés, ayant de 1 à 22 atomes de carbone, tels que les myristates alkyle notamment le myristate de butyle, le myristate de propyle, les palmitates d'alkyle comme le palmitate d'isopropyle, les stéarates d'alkyle notamment le stéarate d'hexadécyle, les oléates d'alkyle, notamment l'oléate de dodécyle, les laurates d'alkyle, notamment le laurate d'hexyle, le dicaprylate de propylène glycol, le cocoate d'éthyl-2-hexyle, les esters de l'acide lactique, de l'acide béhennique, de l'acide isostéarique tel que l'isostéarate d'isostéaryle,
   - et les huiles silicones regroupant les polydiméthylsiloxanes cycliques, les polydiméthylsiloxanes α-ω hydroxylées, les polydiméthylsiloxanes α-ω triméthylsilyllés, les polyorganosiloxanes comme les polyalkylméthylsiloxanes, les polyméthylphénylsiloxanes, les polydiphénylsiloxanes, les dérivés aminés des silicones, les cires silicones, les silicones copolyéthers (comme l'huile SILBIONE 70646® commercialisée par la société RHONE-POULENC ou DC 190® commercialisée par DOW CORNING) ou les dérivés mixtes de silicones comme les copolymères mixtes polyalkylméthylsiloxanes-silicones copolyéthers.
2-b) ou bien l'agent émulsionnant représente de 1 à 2,5 % du poids total de l'émulsion et l'huile est choisie parmi :
   - l'huile d'amandes douces, l'huile de coprah, l'huile de ricin, l'huile de jojoba, l'huile d'olive, l'huile de colza, l'huile d'arachide, l'huile de noisette, l'huile de palme, l'huile de noyau d'abricot, l'huile de calophyllum, l'huile de carthame, l'huile d'avocat, l'huile de pépins de cassis, l'huile de tournesol, l'huile de germes de maïs, l'huile de soja, l'huile de coton, l'huile de luzerne, l'huile d'orge, l'huile de pépins de raisin, l'huile de pavot, l'huile de potimarron, l'huile de sézame, l'huile de seigle, l'huile d'onagre, l'huile de passiflore, des dérivés de ces huiles, les huiles hydrogénées,
   - les huiles d'origine animale (suif, huiles de poisson...),
   - les huiles synthétiques, les poly-α-oléfines,
   - les dérivés de la lanoline,
   - les alcanediols possédant de 2 à 10 atomes de carbone,
   - les alcools de formule R⁴-OH où R⁴ est un radical aliphatique, saturé ou insaturé ayant de 1 à 4 insaturations éthyléniques, linéaire ou ramifié, ayant de 12 à 22 atomes de carbone,
   - les polyéthylène glycols, les polypropylène glycols,
   - les esters gras de formule R⁵-0-CO- R⁶ où R⁵ et R⁴ sont indépendamment l'un de l'autre des radicaux aliphatiques, saturés ou insaturés ayant de 1 à 4 insaturations éthyléniques, linéaires ou ramifiés, ayant de 1 à 22 atomes de carbone, tels que les myristates alkyle notamment le myristate de butyle, le myristate de propyle, les palmitates d'alkyle comme le palmitate d'isopropyle, les stéarates d'alkyle notamment le stéarate d'hexadécyle, les oléates d'alkyle, notamment l'oléate de dodécyle, les laurates d'alkyle, notamment le laurate d'hexyle, le dicaprylate de propylène glycol, le cocoate d'éthyl-2-hexyle, les esters de l'acide lactique, de l'acide béhennique, de l'acide isostéarique tel que l'isostéarate d'isostéaryle,
   - et les huiles silicones regroupant les polydiméthylsiloxanes cycliques, les polydiméthylsiloxanes α-ω hydroxylées, les polydiméthylsiloxanes α-ω triméthylsilyllés, les polyorganosiloxanes comme les polyalkylméthylsiloxanes, les polyméthylphénylsiloxanes, les polydiphénylsiloxanes, les dérivés aminés des silicones, les cires silicones, les silicones copolyéthers (comme l'huile SILBIONE 70646® commercialisée par la société RHONE-POULENC ou DC 190® commercialisée par DOW CORNING) ou les dérivés mixtes de silicones comme les copolymères mixtes polyalkylméthylsiloxanes-silicones copolyéthers.

D'une manière générale, lorsque l'agent émulsionnant représente au moins 2,5 % du poids total de l'émulsion, on préfère, en raison de leur toucher agréable, les émulsions contenant de 10 à 30% d'huile par rapport au poids total de l'émulsion.

Les émulsions de la présente invention peuvent, en outre, contenir un agent émulsionnant complémentaire de l'agent émulsionnant comprenant au moins un alcool gras et un polyglycosides de l'invention, de telle sorte que la quantité totale d'agent émulsionnant soit inférieure à 25% par rapport au poids total de l'émulsion. Cependant, en raison de la très grande efficacité des agents émulsionnants à base d'alcool gras et de polyglycosides de l'invention, il est préférable d'utiliser comme agent émulsionnant, uniquement celui de la présente invention.

D'une façon générale, la quantité totale d'agent émulsionnant sera inférieure à 25% du poids total de l'émulsion et de préférence elle représentera de 2,5 à 6% et plus particulièrement de 2,5 à 4% du poids total de l'émulsion.

Selon une caractéristique avantageuse, liée notamment à leur excellente stabilité et leur fort pouvoir suspensif, on préfère les émulsions comprenant des agents émulsionnants à base de polyglycosides comprenant de 80 à 100% et plus particulièrement de 97 à 100% en poids de polypentosides par rapport aux polyglycosides et de 0 à 20% et plus particulièrement 0 à 3% en poids de polyhexosides.

On entend par émulsion présentant un fort pouvoir suspensif, une émulsion qui permet de maintenir en suspension et bien dispersées, des particules solides présentant une taille moyenne de 50 nm à 500 µm après stockage pendant 3 mois à 45°C et centrifugation à 2000 rpm pendant 3 minutes à 20°C.

Chaque reste d'ose peut être sous forme isomérique α ou β, sous forme L OU D, et sous forme furanosique ou pyranosique. On préfère les hexoses de la série D, notamment le D-glucose, le D-galactose et le D-mannose. Pour les pentoses, on préfère le L-arabinose et le D-xylose présents abondamment dans les hémicelluloses de nombreux végétaux.

En raison de l'abondance du glucose sur le marché des sucres, de préférence, les glucosides représentent au moins 80% des polyhexosides. Pour les mêmes raisons, avantageusement, les polyxylosides représentent 85% et de préférence 98% en poids des polypentosides.

On préfère tout particulièrement les alcools gras de formule ROH ayant de 14 à 22 atomes de carbone et notamment les mélanges d'hexadécanol et d'octadécanol et les mélanges de tétradécanol et d'octadécanol.

De plus, en raison de leur fabrication rapide, on préfère tout particulièrement les compositions de l'invention qui comportent des polyglycosides dont les radicaux R¹ et R² ou le radical R³ sont identiques au radical R de l'alcool gras.

3) En raison de leur efficacité et de leur facilité de fabrication, On préfère tout particulièrement les agents émulsionnants comprenant de 40 à 60% en poids d'alcools gras par rapport au poids total de l'agent émulsionnant et de préférence de 47 à 52% d'alcool gras, le reste étant sauf des impuretés les polyglycosides.

Les agents émulsionnants à base de polyglycosides et d'alcools gras peuvent être préparés par simple mélange de leurs constituants en des proportions telles que mentionnées précédemment. Les techniques d'homogénéisation utilisées sont celles couramment utilisées pour mélanger des constituants solides ou liquides. Pour les constituants solides, on préfère cependant, si cela est possible, les mélanger à une température supérieure à leurs points de fusion sous forme liquide.

Les agents émulsionnants peuvent être préparées selon l'une des deux voies classiquement utilisées pour la synthèse des polyglycosides d'alkyle.

La première voie consiste à mettre directement en contact le sucre réducteur et l'alcool gras en présence d'un catalyseur acide pour obtenir les polyglycosides.

La seconde voie consiste, dans un premier temps, à réaliser la glycosidation avec un alcool court qui répond à la formule R⁷OH, R⁷ étant un radical alkyle ayant de 1 à 5 atomes de carbone. Dans un second temps, on effectue une transglycosidation qui consiste à déplacer l'alcool court de formule R⁷OH par un alcool gras.

Chacune de ces deux voies peut être, le cas échéant, complétée par des opérations de neutralisation, de filtration, d'élimination de l'alcool gras en excès et de décoloration.

Avantageusement, notamment si on travaille à partir de sucres réducteurs cristallisés, on préfère utiliser la première voie directe qui présente l'avantage d'être plus rapide et facile à mettre en oeuvre. Cependant, lorsqu'on utilise des sucres réducteurs sous forme de sirops, il est préférable d'utiliser la seconde voie qui permet d'obtenir un milieu réactionnel plus homogène et par conséquent des polyglycosides de meilleure qualité, ne contenant ou contenant très peu de produits de dégradation.

On entend par sucres réducteurs, les hexoses, les pentoses et les oligosaccharides correspondants présentant un hydroxyle anomère libre.

Lors de la glycosidation directe des sucres par l'alcool gras ou un mélange d'alcool gras, ou lors de la transglycosidation si le greffage est réalisé en deux étapes ; l'alcool gras est utilisé de préférence en excès (1 à 3 et de préférence 1,5 à 2,5 équivalents molaires par rapport aux sucres réducteurs), de telle sorte que le produit de la réaction contienne les quantités précédemment spécifiées d'alcool gras libre et de polyglycosides.

On pourrait également éliminer partiellement ou totalement l'alcool gras ou le mélange d'alcool gras en fin de synthèse, puis ajouter dans des proportions déterminées un alcool gras ou un mélange d'alcool gras différent ou identique à ceux utilisés pendant la synthèse afin d'obtenir l'agent émulsionnant de l'invention.

On préfère cependant la première solution qui consiste à utiliser l'alcool gras en excès de telle sorte que le produit de la réaction contienne les quantités spécifiées d'alcools gras et de polyglycosides.

En pratique, il existe trois principales voies pour obtenir les agents émulsionnants selon l'invention à partir de sucres réducteurs et d'alcools gras.

La première voie consiste à effectuer séparément la glycosidation des sucres réducteurs (hexoses tels que le glucose, le galactose, le mannose et les oligosaccharides correspondants, pentoses choisis parmi l'arabinose et le xylose et les oligosaccharides correspondants) par mise en contact avec un ou plusieurs alcools gras en présence d'un catalyseur acide habituellement utilisé pour les réactions de glycosylation. On préfère mettre l'alcool gras en excès (1 à 3 et de préférence 1,5 à 2,5 équivalents molaires par rapport aux sucres réducteurs) de telle sorte que le produit de la réaction contiennent les quantités spécifiées d'alcool gras libre et de polyglycosides. Après neutralisation du catalyseur acide, on obtient les polypentosides de formule (I) :

R¹O(P)ₙ₁ (I)

et les polyhexosides de formule (II):

R²O(H)ₙ₂ (II)

Ensuite, 66 à 100%, avantageusement 80 à 100% et de préférence 97 à 100% en poids par rapport au poids total des polyglycosides, de polypentosides et 0 à 34%, avantageusement 0 à 20% et de préférence 0 à 3% en poids par rapport au poids total des polyglycosides, de polyhexosides sont mélangés en présence, si cela est nécessaire, d'alcool gras de formule ROH afin d'obtenir les agents émulsionnants de l'invention.

La seconde voie consiste à mélanger 66 à 100%, avantageusement 80 à 100% et de préférence 97 à 100% en poids par rapport au poids total de sucres réducteurs, de pentoses et/ou des oligosaccharides correspondants, avec 0 à 34%, avantageusement 0 à 20% et de préférence 0 à 3% en poids par rapport au poids total de sucres réducteurs, d'hexoses et/ou des oligosaccharides correspondants et d'effectuer la glycosidation du mélange de sucres réducteurs ainsi obtenu. La glycosylation est réalisée en présence d'un catalyseur acide avec un excès (1 à 3 et de préférence 1,5 à 2,5 équivalents molaires par rapport aux sucres réducteurs) d'alcool gras de telle sorte que, de préférence, le produit de la réaction contiennent les quantités spécifiées d'alcools gras libres. Le produits de la réaction est neutralisé et si cela est nécessaire de l'alcool gras de formule ROH est ajouté afin d'obtenir les spécifications précitées.

Enfin, la troisième voie consiste à utiliser des sirops de mélanges de sucres réducteurs dérivés de matières premières végétales riches en amidon et en hémicellulose contenant de 66 à 100%, de préférence 80 à 100% et plus particulièrement 97 à 100% de pentoses et 0 à 34%, de préférence 0 à 20% et plus particulièrement 0 à 3% hexoses et d'effectuer la glycosidation de ces sirops de sucres réducteurs avec des alcools gras afin d'obtenir les agents émulsionnants de l'invention.

Selon une caractéristique avantageuse de l'invention, liée notamment à la simplicité de la synthèse industrielle, à la très forte réactivité des pentoses par rapport à celle du glucose pendant la réaction de glycosylation (voir WO 9729115) et à l'abondance naturelle du D-xylose, on préfère tout particulièrement les émulsions comprenant un agent émulsionnant préparé par la mise en contact directe de D-xylose cristallisé avec 1 à 3 et de préférence 1,5 à 2,5 équivalents molaires par rapport au xylose, d'alcool gras ou un mélange d'alcools gras de formule ROH de telle sorte que le produit de la réaction contienne les quantités précédemment spécifiées d'alcool gras et de polyglycosides en présence d'un catalyseur acide tel que l'acide sulfurique, un acide sulfonique tel que l'acide méthanesulfonique, l'acide chlorhydrique, l'acide hypophosphoreux ou tout autre catalyseur acide permettant d'effectuer une glycosidation et leurs mélanges, à une température comprise entre 50 et 100°C et de préférence entre 80 et 90°C. Le catalyseur acide est ensuite neutralisé. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou alcalino-terreux, notamment la soude, ou une base organique telle que la triéthanolamine. On peut par la suite, si cela est nécessaire, filtrer les insolubles ou évaporer une partie ou la totalité de l'alcool gras libre. L'agent émulsionnant obtenu n'est pas coloré et est exempt de produits de dégradation. Il se présente en fin de synthèse, selon l'alcool gras ou le mélange d'alcools gras utilisé, sous la forme d'une cire solide, pâteuse ou liquide. A partir d'une cire solide, il est possible, notamment pour une utilisation ultérieure plus 5 aisée, d'obtenir de la poudre, des paillettes ou encore des perles ou des pastilles. On préfère isoler la composition sous forme de pastilles hémisphériques d'environ 2 mm de diamètre et 0,7 mm d'épaisseur, très facile à utiliser par la suite, notamment pour la fabrication d'émulsions

Bien entendu, les émulsions de l'invention comprenant un agent émulsionnant contenant au moins un alcool gras et des polyglycosides, peuvent également contenir de 0 à 50% de une ou plusieurs matières actives telles que des adjuvants cosmétiques, lipophiles ou hydrophiles classiques, notamment ceux qui sont déjà utilisés de manière habituelle dans la fabrication et l'obtention d'émulsions. Parmi les adjuvants cosmétiques classiques susceptibles d'être contenus dans la phase aqueuse et/ou la phase grasse des émulsions, on peut citer notamment :
- les épaississants et les gélifiants ioniques, ou non ioniques, comme les dérivés de cellulose (carboxyméthylcellulose, hydroxyéthylcellulose), de guar (hydroxypropylguar, carboxyméthylguar, carboxyméthylhydroxypropylguar...), de caroube, les exsudats d'arbres (gomme arabique, le karaya...), les extraits d'algues marines (alginates, carraghénates...), les exsudats de micro-organismes (gomme de xanthane),
- les agents hydrotropes, comme les alcools courts en C2-C8, en particulier l'éthanol, les diols et glycols comme le diéthylène glycol, dipropylèneglycol,...
- des agents hydratants ou humectants pour la peau comme le glycérol, le sorbitol, le collagène, la gélatine, l'aloe vera, l'acide hyaluronique, l'urée ou des agents protecteurs de la peau, comme les protéines ou hydrolysats de protéines, les polymères cationiques, comme les dérivés cationiques du guar (JAGUAR C13S®, JAGUAR C162®, HICARE 1000® commercialisés par RHONE-POULENC),
- des glycolipides tels que les sophoroses lipides,
- les poudres ou des particules minérales comme du carbonate de calcium, les oxydes minéraux sous forme de poudre ou sous forme colloïdale (particules de taille inférieure ou de l'ordre de un micromètre, parfois de quelques dizaines de nanomètres) comme du dioxyde de titane, de la silice, des sels d'aluminium utilisés généralement comme antitranspirants, du kaolin, du talc, les argiles et leurs dérivés, ...
- les agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisés traditionnellement dans des compositions cosmétiques sont généralement introduits dans ces compositions à hauteur de 0,01 à 3% en poids.
Au lieu de ces agents chimiques, on peut parfois utiliser des agents modifiant l'activité de l'eau et augmentant fortement la pression osmotique comme les carbohydrates ou des sels.
- les filtres solaires organiques actifs dans l'UV-A et/ou l'UV-B pour protéger la peau ou les cheveux des agressions du soleil et des rayons UV, comme les composés autorisés dans la directive Européenne N° 76/768/CEE, ses annexes et les modifications ultérieures de cette directive,
- les monopigments minéraux photoprotecteurs, comme le dioxyde de titane ou les oxydes de cérium sous forme de poudre ou de particules colloïdales.
- les adoucissants, les antioxydants, les agents autobronzant comme la DHA, les agents répulsifs contre les insectes, les vitamines, les parfums, les charges, les séquestrants, les colorants, les agents tampon...
- les abrasifs tels que les noyaux d'abricots broyés, les microbilles...

Les émulsions préparées à partir de l'agent émulsionnant de l'invention peuvent être utilisées dans différentes applications cosmétiques ou dermatologiques, par exemple sous forme de crèmes pour le visage, pour le corps, pour le cuir chevelu ou pour la chevelure ou sous forme de lait pour le corps ou pour le démaquillage ou encore sous forme de pommades par exemple à usage pharmaceutique. Ces émulsions peuvent également être utilisées pour le maquillage, notamment sous forme de fonds de teint, après addition de pigments. Elles peuvent aussi être utilisées comme crèmes solaires après addition de filtres UVA et/ou UVB et/ou DHA, ou comme crèmes ou laits après soleil après addition de composés apaisants tels que le panthénol.

Les émulsions peuvent également contenir des agents tensioactifs lavants, moussants ou détergents ioniques ou non ioniques tels que le lauryléther sulfate de sodium, les alkyl-bétaïnes, les APG... pour faire des émulsions lavantes telles que les crèmes lavantes hydratantes, ou des émulsions pour le rasage.

Les émulsions peuvent contenir en outre, en vue d'augmenter leurs qualités cosmétiques, une cire cosmétique telle que par exemple de la cire de riz, de la cire de candellila, de la cire du Japon. La proportion de cire est généralement comprise entre 0,5 et 3%, et de préférence entre 1 et 2% en poids par rapport au poids total de l'émulsion.

Les agents émulsionnants à base de polyglycosides et d'alcools gras de l'invention peuvent être utilisés également dans des formulations où il est nécessaire de maintenir en suspension dans l'eau des solides finement divisés, comme les formulations de matières actives agrochimiques (herbicides, insecticides, fongicides,...) connues sous le nom générique de "suspensions concentrées". Outre un agent dispersant ou émulsionnant utilisé de 1 à 50 g/l, on retrouve comme additifs dans une formulation de suspension concentrée, des additifs comme ceux décrits dans la brochure commerciale "Auxiliaries for agrochemical formulations" éditées par RHONE-POULENC GERONAZZO SpA. On peut citer par exemple un tensioactif mouillant, pris parmi les dérivés alcoylés d'alcools arylaliphatiques, les dérivés aryl sulfonés comme l'isopropylnaphtalène sulfonate de sodium, commercialisé sous le nom SUPRAGIL WP® par RHONE-POULENC GERONAZZO, les dialkyl sulfosuccinates comme le di-éthyl-2-hexyl sulfosuccinate de sodium, des polymères dispersants, comme les acides polyacryliques et leurs sels, les copolymères anhydride (ou acide) maléique - diisobutylène et leurs sels comme le GEROPON T36® (RHONE-POULENC GERONAZZO), les méthylnaphtalène sulfonates de sodium condensés comme le SUPRAGIL MNS90® (RHONE-POULENC GERONAZZO), les polymères dispersants dérivés de la lignine comme les lignosulfonates de sodium ou de calcium ou d'autres tensioactifs dispersants comme les dérivés alcoxylés, éventuellement sulfatés ou phosphatés de tristyrylphénols. On peut trouver en outre dans ces formulations, des additifs antigels comme le propylène glycol et des additifs épaississants, modifiant le comportement rhéologique de la suspension comme la gomme xanthane, les dérivés de la cellulose (carboxyméthylcellulose), la gomme guar ou ses dérivés, des argiles ou des argiles modifiées comme la bentonite et les bentones. Parmi les matières actives pouvant être formulées ainsi, on trouve généralement celles dont le point de fusion est supérieur à 45°C préférentiellement supérieur à 60°C et dont la solubilité dans l'eau est inférieure à 10 g/l, préférentiellement inférieure à 1 g/l. Les matières actives phytosanitaires concernées sont les herbicides, les fongicides et insecticides, tels que ceux décrits dans THE PESTICIDE MANUAL (9° édition, C.R. WORKLING et R.J. HANCE, éditeurs, publié par The British Crop Protection Council) et répondant aux critères ci-dessus.

Les agents émulsionnants à base de polyglycosides et d'alcools gras de l'invention peuvent être utilisés également dans des formulations phytosanitaires comme les concentrées émulsionnables, les suspensions concentrées, les émulsions concentrées, les microémulsions ou les suspoémulsions. On les utilise alors à des concentrations comprises entre 5 et 150 g/l. Les matières actives phytosanitaires peuvent être celles décrites précédemment pour les suspensions concentrées. Les agents émulsionnants de la présente invention sont particulièrement efficaces pour la préparation d'émulsions concentrées phytosanitaires. La matière active est mise en suspension dans l'eau grâce à l'agent émulsionnant, qui assure la stabilité du concentré au stockage ainsi que la spontanéité et la stabilité de l'émulsion diluée lors de l'utilisation. Les compositions typiques sont constituées d'environ 40 à 80% de matière active comme par exemple les esters d'acides gras tels que les esters méthyliques de colza (EMC) commercialisés par Robbe et de 1 à 15% d'additifs. Cette présentation offre les facilités d'emploi des concentrés émulsionnables, avec l'avantage majeur d'un véhicule aqueux à la place d'un solvant, ce qui assure une bonne sécurité.

Les agents émulsionnants de la présente invention sont également efficaces pour la préparation d'émulsions de gasoil utilisées comme carburant diesel. Elles permettent d'incorporer de l'eau dans le carburant et de réduire ainsi les émissions de gaz de combustion toxiques. Ces émulsions sont généralement constituées de 60 à 97% de gasoil, de 2,5 à 39,5% d'eau et de 0.5 à 5% d'agent émulsionnant. Elles peuvent également contenir les additifs utilisés couramment dans les carburants de type diesel.

Quatre principales méthodes de fabrication des émulsions sont proposées :
- La première consiste à chauffer tous les ingrédients en même temps à une température comprise entre 50 et 90°C et en particulier entre 70 et 75°C, puis à homogénéiser avec un agitateur rotatif à pâle tournant de 500 à 15000 rpm, en particulier de 1000 à 2000 rpm, à une température comprise entre 50 et 90°C et enfin à refroidir sous agitation lente (de 100 à 1000, en particulier de 300 à 500 rpm) jusqu'à une température de l'ordre de 25°C. Dans le cas où l'homogénéisation est vive à chaud, il n'est pas toujours utile d'agiter l'émulsion pendant son refroidissement.
- La seconde consiste à travailler par inversion de phase. Dans ce cas, les phases lipophile et hydrophile sont chauffées séparément à une température comprise entre 50 et 90°C. La phase lipophile, qui contient la composition de l'invention, est mise sous agitation vive avec un agitateur rotatif à pâle tournant de 500 à 15000 rpm, en particulier de 1000 à 2000 rpm et on ajoute lentement, à un débit tel que la phase hydrophile est instantanément absorbée par la phase lipophile, à cette phase la phase hydrophile jusqu'à l'inversion de phase caractérisée par un changement brutal de la viscosité L'addition peut être ensuite plus rapide à un débit tel que la phase hydrophile stagne 1 à 3 secondes au dessus de la phase lipophile si l'ajout a lieu par le dessus. On laisse enfin refroidir l'émulsion sous agitation lente (de 100 à 1000, en particulier de 300 à 500 rpm) jusqu'à une température de l'ordre de 25°C.
- La troisième méthode est réalisée par dispersion. Dans ce cas les phases lipophile (qui contient l'agent émulsionnant de l'invention) et hydrophile sont chauffées séparément à une température comprise entre 50 et 90°C et de préférence entre 70 et 75°C. La phase hydrophile est mise sous agitation avec un agitateur rotatif à pâle tournant de 500 à 15000 rpm, en particulier de 1000 à 2000 rpm et on y ajoute progressivement, à un débit tel que la phase lipophile est instantanément absorbée par la phase hydrophile, la phase lipophile. On laisse ensuite refroidir l'émulsion sous agitation lente ( de 100 à 1000, en particulier de 300 à 500 rpm) jusqu'à une température de l'ordre de 25°C.
- La quatrième méthode consiste à chauffer tous les ingrédients en même temps à une température comprise entre 50 et 90°C, puis à homogénéiser avec un agitateur rotatif à pâle tournant de 100 à 1000 rpm et à passer le mélange obtenu une ou plusieurs fois dans un homogénéisateur « haute pression ». Les homogénéisateurs « haute pression » sont des appareils permettant de soumettre le mélange à émulsionner à des pressions variant de 10 à 1000 bars et en particulier de 50 à 500 bars.

Selon un autre aspect de l'invention, la composition à base de polyglycosides peut être utilisée comme base auto-émulsionnable pour la préparation d'émulsions par dispersion à chaud des compositions de l'invention, par exemple entre environ 50 et 90 °C, dans de l'eau ou un milieu polaire approprié, par simple agitation, notamment mécanique ou par sonication. Si on disperse la composition dans l'eau par agitation ou par sonication à une température proche du point de fusion de l'agent émulsionnant, on obtient des dispersions riches en vésicules.

Les deux principaux critères d'évaluation qui permettent de démontrer les bonnes stabilités des émulsions obtenues avec les agents émulsionnants de la présente invention sont les suivantes
- La résistance à la centrifugation par la détermination de l'indice de crémage, qui est défini comme le pourcentage en volume d'émulsion crémée résiduelle après un traitement de déstabilisation par centrifugation à 4080 g pendant 1 heure à 25°C. Il faut considérer que le résultat est d'autant meilleur que le pourcentage d'émulsion crémée est extrême. Après centrifugation, l'indice de crémage est le rapport du volume d'émulsion résiduelle sur le volume total d'émulsion initiale multiplié par 100.
- Le suivi de la rétrodiffusion des émulsions au moyen d'un analyseur macroscopique à balayage vertical tel que le « TURBISCAN 2000 ». En effet, l'instabilité des émulsions est souvent le résultat d'une conjonction de processus physiques impliquant notamment deux grands types de phénomènes :
- L'augmentation de la taille des gouttelettes ou des agrégats (coalescence, floculation).
- La migration des gouttelettes au sein de l'échantillon (crémage, sédimentation).
Le crémage et la sédimentation sont parfois considérés comme des phénomènes anodins alors que la coalescence est toujours catastrophique pour le formulateur en raison de son irréversibilité. Il apparaît donc important de détecter très tôt ces phénomènes (pour réduire les tests de vieillissement qui sont au moins de 90 jours à 45°C) et de les identifier. La méthode d'évaluation de la stabilité des émulsions proposée ici permet de détecter des variations locales de concentrations et/ou de tailles des particules en milieu concentré et ceci bien avant l'oeil nu. Cette technique nous permet en plus de nous renseigner sur le ou les types de processus physiques impliqués dans la déstabilisation du mélange ainsi que sur leur ordre d'apparition. Les émulsions les plus stables sont celles qui présentent une variation de la rétrodiffusion en fonction du temps la plus faible.

Pour évaluer les bons pouvoirs suspensifs des émulsions de la présente invention, nous les avons préparées en ajoutant comme additif des particules solides de tailles comprises entre 0,05 et 0,250 mm. Nous avons ensuite soumis les émulsions à un traitement de déstabilisation par centrifugation à 2000 rpm pendant 3 minutes à 20°C. Nous avons alors mesuré le culot de particules solides relarguées au fond du tube. Les résultats sont exprimés en pourcentage de culot défini par le volume de culot par rapport au volume total d'émulsion centrifugée. Les émulsions qui présentent les meilleurs pouvoirs suspensifs sont celles pour lesquelles nous n'obtenons pas du tout de culot.

Les exemples suivants ont pour but d'illustrer la présente invention.

### Exemple 1 de synthèse

### Procédé de préparation de polyglucosides de tétradécyle et d'octadécyle

140 g de D-glucose anhydre sont mis en suspension dans 371 g d'alcool gras (tétradécanol : 25%, octadécanol : 75%) contenant 2,80 g d'acide sulfurique. Le milieu réactionnel est maintenu sous pression réduite pendant 3 heures à 100°C. Après filtration, l'acidité du milieu est neutralisée par une solution aqueuse de soude à 30,5% jusqu'à pH 7 à 8. Le produit de la réaction appelé agent émulsionnant à base de glucosides de tétradécyle et d'octadécyle (486 g) se présente sous la forme d'une pâte solide qui contient 50,5% en poids d'alcool gras résiduel. Il est broyé afin d'obtenir une poudre de granulométrie inférieure à 800 µm.

### Exemple 2 de synthèse

### Procédé de préparation de polyglucosides de cétéaryle

140 g de D-glucose anhydre sont mis en suspension dans 377,5 g d'alcool gras (hexadécanol : 33%, octadécanol : 67%) contenant 2,80 g d'acide sulfurique. Le milieu réactionnel est maintenu sous pression réduite pendant 3 heures à 100°C. Après filtration, l'acidité du milieu est neutralisée par une solution aqueuse de soude à 30,5% jusqu'à pH 7 à 8. Le produit de la réaction appelé agent émulsionnant à base de glucosides cétéaryle (492,6 g) se présente sous la forme d'une pâte solide qui contient 50,7% en poids d'alcool gras résiduel. Il est broyé afin d'obtenir une poudre de granulométrie inférieure à 800 µm.

### Exemple 3 de synthèse

### Procédé de préparation de polyglucosides de cétéaryle

100 g de D-glucose anhydre sont mis en suspension dans 264,8 g d'alcool gras (hexadécanol : 50%, octadécanol : 50%) contenant 2 g d'acide sulfurique. Le milieu réactionnel est maintenu sous pression réduite pendant 3 heures à 100°C. Après filtration, l'acidité du milieu est neutralisée par une solution aqueuse de soude à 30,5% jusqu'à pH 7 à 8. Le produit de la réaction appelé agent émulsionnant à base de glucosides cétéaryle (335 g) se présente sous la forme d'une pâte solide qui contient 50,5% en poids d'alcool gras résiduel. Celui-ci est ramené ensuite à 55% par rapport à l'ensemble du produit de la réaction par addition d'un mélange d'hexadécanol et d'octadécanol (50/50) et est broyé afin d'obtenir une poudre de granulométrie inférieure à 800 µm.

### Exemple 1

### Procédé de préparation d'un agent émulsionnant à base de polyxylosides et d'alcool gras selon l'invention

140 g de D-xylose anhydre à 97 % de pureté sont mis en suspension dans 478 g d'alcool gras (tétradécanol : 25%, octadécanol : 75%) contenant 4,2 g d'acide sulfurique. Le milieu réactionnel est maintenu sous pression réduite pendant 3 heures à 90°C. Après filtration, l'acidité du milieu est neutralisée par une solution aqueuse de soude à 30,5% jusqu'à pH 7 à 8. Le produit de la réaction appelé agent émulsionnant à base de polyxylosides d'alkyle (590 g) se présente sous la forme d'une pâte solide qui contient 49,5% en poids d'alcool gras résiduel. Il est broyé afin d'obtenir une poudre de granulométrie inférieure à 800 µm.

### Exemple 2

### Procédé de préparation d'un agent émulsionnant à base de polyxylosides et d'alcool gras selon l'invention

100 g de D-xylose anhydre à 97 % de pureté sont mis en suspension dans 340,6 g d'alcool gras (hexadécanol : 33%, octadécanol : 67%) contenant 3,0 g d'acide sulfurique. Le milieu réactionnel est maintenu sous pression réduite pendant 1,5 heures à 90°C. Après filtration, l'acidité du milieu est neutralisée par une solution aqueuse de soude à 30,5% jusqu'à pH 7 à 8. Le produit de la réaction appelé agent émulsionnant à base de polyxylosides d'alkyle (408 g) se présente sous la forme d'une pâte solide qui contient 53% en poids d'alcool gras résiduel. Il est broyé afin d'obtenir une poudre de granulométrie inférieure à 800 µm.

### Exemple 3

### Procédé de préparation d'un agent émulsionnant à base de polyxylosides de cétéaryle et d'alcool gras selon l'invention

140 g de D-xylose anhydre à 97 % de pureté sont mis en suspension dans 488 g d'alcool gras (hexadécanol : 30%, octadécanol : 70%) contenant 4,2 g d'acide sulfurique. Le milieu réactionnel est maintenu sous pression réduite pendant 3 heures à 90°C. Après filtration, l'acidité du milieu est neutralisée par une solution aqueuse de soude à 30,5% jusqu'à pH 7 à 8. Le produit de la réaction appelé agent émulsionnant à base de polyxylosides de cétéaryle (600 g) se présente sous la forme d'une pâte solide qui contient 50% en poids d'alcool gras résiduel. Il est broyé afin d'obtenir une poudre de granulométrie inférieure à 800 µm.

### Exemple 4

### Procédé de préparation d'un agent émulsionnant à base de polyxylosides et d'alcool gras selon l'invention

140 g de D-xylose anhydre à 97 % de pureté sont mis en suspension dans 478 g d'alcool gras (hexadécanol : 30%, octadécanol : 70%) contenant 4,2 g d'acide sulfurique. Le milieu réactionnel est maintenu sous pression réduite pendant 1,5 heures à 90°C. Après filtration, l'acidité du milieu est neutralisée par une solution aqueuse de soude à 30,5% jusqu'à pH 7 à 8. Le produit de la réaction appelé agent émulsionnant à base de polyxylosides d'alkyle (572 g) se présente sous la forme d'une pâte solide qui contient 50% en poids d'alcool gras résiduel. Il est broyé afin d'obtenir une poudre de granulométrie inférieure à 800 µm.

### Exemple 5

### Procédé de préparation de polyarabinosides de tétradécyle et d'octadécyle

100 g de L-arabinose anhydre sont mis en suspension dans 318 g d'alcool gras (tétradécanol : 25%, octadécanol : 75%) contenant 2,00 g d'acide sulfurique. Le milieu réactionnel est maintenu sous pression réduite pendant 1,5 heures à 90°C. . Après filtration, l'acidité du milieu est neutralisée par une solution aqueuse de soude à 30,5% jusqu'à pH 7 à 8. Le produit de la réaction appelé agent émulsionnant à base d'arabinosides de tétradécyle et d'octadécyle (395 g) se présente sous la forme d'une pâte solide qui contient 44,5% en poids d'alcool gras résiduel. Celui-ci est ramené ensuite à 50% par rapport à l'ensemble du produit de la réaction par addition d'un mélange de tétradécanol et d'octadécanol (25/75) et est broyé afin d'obtenir une poudre de granulométrie inférieure à 800 µm.

### Exemple 6

### Procédé de préparation de polyarabinosides de cétéaryle

100 g de L-arabinose anhydre sont mis en suspension dans 324 g d'alcool gras (hexadécanol : 33%, octadécanol : 67%) contenant 2,00 g d'acide sulfurique. Le milieu réactionnel est maintenu sous pression réduite pendant 3 heures à 80°C. Après filtration, l'acidité du milieu est neutralisée par une solution aqueuse de soude à 30,5% jusqu'à pH 7 à 8. Le produit de la réaction appelé agent émulsionnant à base d'arabinosides de cétéaryle (401 g) se présente sous la forme d'une pâte solide qui contient 44,7% en poids d'alcool gras résiduel. Celui-ci est ramené ensuite à 50% par rapport à l'ensemble du produit de la réaction par addition d'un mélange d'hexadécanol et d'octadécanol (33/67) et est broyé afin d'obtenir une poudre de granulométrie inférieure à 800 µm.

### Exemple 7

### Procédé de préparation d'un agent émulsionnant à base de polyglycoside et d'alcool gras selon l'invention

350 g de sirop de sucres contenant 87,5 g d'eau, 190 g de D-xylose, 36 g de L-arabinose, 34,5 g de D-glucose et 2 g de D-galactose sont ajoutés goutte à goutte en 1 heure 30 dans 394 g de *n*-butanol contenant 5,2 g d'acide sulfurique et 54 g d'eau à une température de l'ordre de 100 à 105°C. L'eau est éliminée au cours de la réaction par distillation azéotropique du mélange eau/butanol. On ajoute ensuite 45 % du milieu réactionnel obtenu à 366 g d'alcool gras constitué de 25% de tétradécanol et 75% d'alcool gras dérivé de colza (mélange d'hexadécanol : 10% et d'octadécanol :90 % en poids) contenant 1,2 g d'acide sulfurique, à une température de 90°C. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après refroidissement à 20°C et broyage du solide obtenu, on recueille 480 g d'agent émulsionnant qui contient 48 % en poids par rapport au poids total de la composition d'alcool gras.

### Exemple 8

### Procédé de préparation d'un agent émulsionnant à base de polyglycoside et d'alcool gras selon l'invention

350 g de sirop de sucres contenant 87,5 g d'eau, 190 g de D-xylose, 36 g de L-arabinose, 34,5 g de D-glucose et 2 g de D-galactose sont ajoutés goutte à goutte en 1 heure 30 dans 393,5 g de *n*-butanol contenant 5,2 g d'acide sulfurique et 53,5 g d'eau à une température de l'ordre de 100 à 105°C. L'eau est éliminée au cours de la réaction par distillation azéotropique du mélange eau/butanol. On ajoute ensuite le milieu réactionnel obtenu à 837 g d'alcool gras constitué de 30% d'hexadécanol et 70% d'octadécanol contenant 2,6 g d'acide sulfurique, à une température de 90°C. Le butanol est éliminé sous pression réduite en continu pendant l'addition. Ensuite, à la même température, l'acidité du milieu est neutralisée par une solution aqueuse de soude à 30,5 % jusqu'à pH 7 à 8 et le produit est décolorée en présence d'eau oxygénée. Après pastillage, on recueille 1061 g d'agent émulsionnant qui contient 49 % en poids par rapport au poids total de la composition d'alcool gras.

### Exemple 9

### Procédé de préparation d'un agent émulsionnant à base de polyglycoside et d'alcool gras selon l'invention

20 g de l'agent émulsionnant à base de polyglucoside de tétradécyle et d'octadécyle de l'exemple 1 de synthèse, 68 g de l'agent émulsionnant à base de polyxylosides de tétradécyle et d'octadécyle de l'exemple 1 et 12 g de l'agent émulsionnant à base de polyarabinosides de tétradécyle et d'octadécyle de l'exemple 5, sont mélangés afin d'obtenir 100 g d'agent émulsionnant à base de polyglycosides contenant environ 50 % en poids d'alcool gras, le reste étant constitué de polyglycosides de tétradécyle et d'octadécyle comprenant 80% en poids, par rapport aux polyglycosides, de polypentosides (85% en poids de polyxylosides et 15 % en poids de polyarabinosides par rapport aux polypentosides) et 20 % en poids de polyhexosides.

### Exemple 10

### Procédé de préparation d'un agent émulsionnant à base de polyglycoside et d'alcool gras selon l'invention

34 g de l'agent émulsionnant à base de polyglucoside de cétéaryle de l'exemple 2 de synthèse, 56 g de l'agent émulsionnant à base de polyxylosides de cétéaryle de l'exemple 2 et 10 g de l'agent émulsionnant à base de polyarabinosides de cétéaryle de l'exemple 6, sont mélangés afin d'obtenir 100 g d'agent émulsionnant à base de polyglycosides contenant environ 51 % en poids d'alcool gras, le reste étant constitué de polyglycosides de cétéaryle comprenant 66% en poids, par rapport aux polyglycosides, de polypentosides (85% en poids de polyxylosides et 15 % en poids de polyarabinosides par rapport aux polypentosides) et 34 % en poids de polyhexosides.

### Exemple 11

### Procédé de préparation d'un agent émulsionnant à base de polyglycoside et d'alcool gras selon l'invention

20 g de l'agent émulsionnant à base de polyglucoside de tétradécyle et d'octadécyle de l'exemple 1 de synthèse et 180 g de l'agent émulsionnant à base de polyxylosides de tétradécyle et d'octadécyle de l'exemple 1, sont mélangés afin d'obtenir 200 g d'agent émulsionnant à base de polyglycosides contenant environ 50 % en poids d'alcool gras, le reste étant constitué de polyglycosides de tétradécyle et d'octadécyle comprenant 90% en poids, par rapport aux polyglycosides de polypentosides (polyxylosides) et 10 % en poids de polyhexosides (polyglucosides).

### Exemple 12

### Procédé de préparation d'un agent émulsionnant à base de polyglycoside et d'alcool gras selon l'invention

115 g de D-xylose, 20 g de L-arabinose et 15 g de D-glucose sont placés dans 235 g d'éthahol à 96% contenant 3 g d'acide sulfurique. Le milieu est porté à reflux pendant 3 heures. On ajoute ensuite le milieu réactionnel obtenu à 583 g d'alcool gras constitué de 30% d'hexadécanol et 70% d'octadécanol contenant 1,5 g d'acide sulfurique, à une température de 90°C. L'éthanol est éliminé sous pression réduite en continu pendant l'addition. Ensuite, à la même température, l'acidité du milieu est neutralisée par une solution aqueuse de soude à 30,5 % jusqu'à pH 7 à 8. Après pastillage, on recueille 701 g d'agent émulsionnant qui contient 58 % en poids par rapport au poids total de la composition d'alcool gras.

### Exemple 13

### Procédé de préparation d'un agent émulsionnant à base de polyglycoside et d'alcool gras selon l'invention

30 g de l'agent émulsionnant à base de polyglucoside de tétradécyle et d'octadécyle de l'exemple 1 de synthèse et 70 g de l'agent émulsionnant à base de polyxylosides de tétradécyle et d'octadécyle de l'exemple 1, sont mélangés afin d'obtenir 100 g d'agent émulsionnant à base de polyglycosides contenant environ 50 % en poids d'alcool gras, le reste étant constitué de polyglycosides de tétradécyle et d'octadécyle comprenant 70% en poids, par rapport aux polyglycosides de polypentosides (polyxylosides) et 30 % en poids de polyhexosides (polyglucosides).

### Exemple 14

### Procédé de préparation d'un agent émulsionnant à base de polyglycosides et d'alcool gras selon l'invention

91 g de D-xylose, 56 g de L-arabinose et 53,9 g de D-glucose monohydraté sont mis en suspension dans 803 g d'alcool gras (hexadécanol : 50%, octadécanol : 50%) contenant 6 g d'acide sulfurique. Le milieu réactionnel est maintenu sous pression réduite pendant 2 heures à 100°C. Après filtration, l'acidité du milieu est neutralisée par une solution aqueuse de soude à 30,5% jusqu'à pH 7 à 8. Le produit de la réaction appelé agent émulsionnant à base de polyglycosides d'alkyle (960 g) se présente sous la forme d'une pâte solide qui contient 56% en poids d'alcool gras résiduel. Il est broyé afin d'obtenir une poudre de granulométrie inférieure à 800 µm.

### Exemple 15

### Procédé de préparation d'un agent émulsionnant à base de polyglycosides et d'alcool gras selon l'invention

200 g de sirop de sucres contenant 25% d'eau, 108 g de D-xylose, 21 g de L-arabinose, 20 g de D-glucose et 1 g de D-galactose sont ajoutés goutte à goutte en 1 heure dans 208 g de *n*-butanol contenant 3 g d'acide sulfurique et 14 g d'eau à une température de l'ordre de 100 à 105°C. L'eau est éliminée au cours de la réaction par distillation azéotropique du mélange eau/butanol. On ajoute ensuite le milieu réactionnel obtenu à 423 g d'alcool gras constitué de 80% de tétradécanol et 20% d'octadécanol contenant 1,5 g d'acide sulfurique, à une température de 90°C. Le butanol est éliminé sous pression réduite en continu pendant l'addition. Ensuite, à la même température, l'acidité du milieu est neutralisée par une solution aqueuse de soude à 30,5 % jusqu'à pH 7 à 8. Après refroidissement à 20°C et broyage du solide obtenu, on recueille 572 g d'agent émulsionnant qui contient 47,5 % en poids par rapport au poids total de la composition d'alcool gras.

### Exemple 16

### Procédé de préparation d'un agent émulsionnant à base de polyglycosides et d'alcool gras selon l'invention

200 g de sirop de sucres contenant 25% d'eau, 108 g de D-xylose, 21 g de L-arabinose, 20 g de D-glucose et 1 g de D-galactose sont ajoutés goutte à goutte en 1 heure dans 208 g de *n*-butanol contenant 3 g d'acide sulfurique et 14 g d'eau à une température de l'ordre de 100 à 105°C. L'eau est éliminée au cours de la réaction par distillation azéotropique du mélange eau/butanol. On ajoute ensuite le milieu réactionnel obtenu à 432 g d'alcool gras constitué de 60% de tétradécanol et 40% d'octadécanol contenant 1,5 g d'acide sulfurique, à une température de 90°C. Le butanol est éliminé sous pression réduite en continu pendant l'addition. Ensuite, à la même température, l'acidité du milieu est neutralisée par une solution aqueuse de soude à 30,5 % jusqu'à pH 7 à 8. Après refroidissement à 20°C et broyage du solide obtenu, on recueille 582 g d'agent émulsionnant qui contient 47 % en poids par rapport au poids total de la composition d'alcool gras.

### Exemple 17

### Procédé de préparation de polyxylosides de cétéaryle

100 g de D-xylose sont mis en suspension dans 318 g d'alcool gras (hexadécanol : 50%, octadécanol : 50%) contenant 2 g d'acide sulfurique. Le milieu réactionnel est maintenu sous pression réduite pendant 3 heures à 80°C. Après filtration, l'acidité du milieu est neutralisée par une solution aqueuse de soude à 30,5% jusqu'à pH 7 à 8. Le produit de la réaction appelé agent émulsionnant à base de polyxylosides de cétéaryle (400 g) se présente sous la forme d'une pâte solide qui contient 43% en poids d'alcool gras résiduel. Celui-ci est ramené ensuite à 55% par rapport à l'ensemble du produit de la réaction par addition d'un mélange d'hexadécanol et d'octadécanol (50/50) et est broyé afin d'obtenir une poudre de granulométrie inférieure à 800 µm.

### Exemple 18

### Exemple d'émulsion fluide selon l'invention

2 g de l'agent émulsionnant de l'exemple 1 sont mis en suspension dans 48 g d'eau osmosée. Le mélange est porté à 50 °C et est ensuite agité (500 rpm) pendant 2 minutes. L'émulsion ainsi formée est ensuite refroidie à température ambiante. Cette émulsion reste stable pendant 3 mois dans une étuve à 45°C.

### Exemple 19

### Exemple d'émulsion fluide selon l'invention

1,5 g de l'agent émulsionnant de l'exemple 2 sont mis en suspension dans 48,5 g d'eau osmosée. Le mélange est porté à 50 °C et est ensuite agité (500 rpm) pendant 2 minutes. L'émulsion ainsi formée est ensuite refroidie à température ambiante. Cette émulsion reste stable pendant 3 mois dans une étuve à 45°C.

### Exemple 20

### Exemple d'émulsion selon l'invention - préparation d'une crème

3 g de l'agent émulsionnant de l'exemple 1 sont mis en suspension dans 47 g d'eau osmosée. Le mélange est porté à 50 °C et est ensuite agité (500 rpm) pendant 2 minutes. L'émulsion ainsi formée est ensuite refroidie à température ambiante. Cette émulsion reste stable pendant 3 mois dans une étuve à 45°C.

### Exemple 21

### Exemple d'émulsion fluide selon l'invention

Chauffer 2 g de l'agent émulsionnant à base de polyglycosides de l'exemple 1, 5 g d'huile de tournesol et 43 g d'eau osmosée en même temps à une température de 70°C, puis homogénéiser (1300 rpm) à la même température pendant 1 minute et enfin refroidir sous agitation lente (300 rpm) jusqu'à une température de l'ordre de 25°C. Après 1 jour de mûrissement, la viscosité de l'émulsion ainsi formée est de 6130 cp (BROOKFIELD DV II - 25°C - 12 rpm - module 3). Cette émulsion reste stable pendant 3 mois dans une étuve à 45°C.

### Exemple 22

### Exemple d'émulsion selon l'invention - préparation d'une crème

Chauffer 3 g de l'agent émulsionnant à base de polyglycosides de l'exemple 7, 15 g d'isostéarate d'isostéaryle et 32 g d'eau osmosée en même temps à une température de 70°C, puis homogénéiser (1300 rpm) à la même température pendant 1 minute et enfin refroidir sous agitation lente (300 rpm) jusqu'à une température de l'ordre de 25°C. Après 1 jour de mûrissement, la viscosité de l'émulsion ainsi formée est de 20000-25000 cp (BROOKFIELD DV II - 25°C - 12 rpm - module 3). Cette émulsion reste stable pendant 3 mois dans une étuve à 45°C.

### Exemple 23

### Exemple d'émulsion fluide selon l'invention

La phase lipophile (10 g d'isostéarate d'isostéaryle) qui contient 4 g d'agent émulsionnant à base de polyglycosides de l'exemple 7 et la phase hydrophile (86 g d'eau osmosée) sont chauffées séparément à une température de 70°C. La phase lipophile est mise sous agitation vive (800 rpm) et on y ajoute en 2 minutes la phase hydrophile jusqu'à l'inversion de phase caractérisée par un changement brutal de la viscosité. L'addition peut être ensuite plus rapide (1 minute). On laisse enfin refroidir l'émulsion sous agitation lente (300 rpm) jusqu'à une température de l'ordre de 25°C.

Après 1 jour de mûrissement, la viscosité de l'émulsion ainsi formée est de 6960 cp (BROOKFIELD DV II - 25°C - 12 rpm - module 3). Cette émulsion reste stable pendant 3 mois dans une étuve à 45°C.

### Exemple 24

### Exemple d'émulsion selon l'invention - préparation d'une crème

La phase lipophile (10 g de miglyol) qui contient 4 g d'agent émulsionnant à base de polyglycosides de l'exemple 8 et la phase hydrophile (86 g d'eau osmosée) sont chauffées séparément à une température de 70°C. La phase lipophile est mise sous agitation vive (800 rpm) et on y ajoute en 2 minutes la phase hydrophile jusqu'à l'inversion de phase caractérisée par un changement brutal de la viscosité. L'addition peut être ensuite plus rapide (1 minute). On laisse enfin refroidir l'émulsion sous agitation lente (300 rpm) jusqu'à une température de l'ordre de 25°C. Après 1 jour de mûrissement, la viscosité de l'émulsion ainsi formée est de 11320 cp (BROOKFIELD DV II - 25°C - 12 rpm - module 3). Cette émulsion reste stable pendant 3 mois dans une étuve à 45°C.

### Exemple 25

### Exemple d'émulsion fluide selon l'invention

La phase lipophile (10 g d'un mélange d'huile de tournesol : 50% et de diméthicone : 50%) qui contient 4 g d'agent émulsionnant à base de polyglycosides de l'exemple 11 et la phase hydrophile (86 g d'eau osmosée) sont chauffées séparément à une température de 70°C. La phase lipophile est mise sous agitation vive (800 rpm) et on y ajoute en 2 minutes la phase hydrophile jusqu'à l'inversion de phase caractérisée par un changement brutal de la viscosité. L'addition peut être ensuite plus rapide (1 minute). On laisse enfin refroidir l'émulsion sous agitation lente (300 rpm) jusqu'à une température de l'ordre de 25°C. Cette émulsion reste stable pendant 3 mois dans une étuve à 45°C.

### Exemple 26

### Exemple d'émulsion fluide selon l'invention

La phase lipophile (10 g d'isostéarate d'isostéaryle) qui contient 4 g d'agent émulsionnant à base de polyglycosides de l'exemple 1 et la phase hydrophile (86 g d'eau osmosée), sont chauffées séparément à une température de 70°C. La phase hydrophile est mise sous agitation (1500 rpm) et on y ajoute progressivement la phase lipophile. On laisse ensuite refroidir l'émulsion sous agitation lente (300 rpm) jusqu'à une température de l'ordre de 25°C. Après 1 jour de mûrissement, la viscosité de l'émulsion ainsi formée est de 6130 cp (BROOKFIELD DV II - 25°C - 12 rpm - module 3). Cette émulsion reste stable pendant 3 mois dans une étuve à 45°C.

### Exemple 27

### Exemple d'émulsion selon l'invention - préparation d'une crème

Chauffer 30 g de l'agent émulsionnant à base de polyglycosides de l'exemple 3, 100 g d'isostéarate d'isostéaryle et 870 g d'eau osmosée en même temps à une température de 70°C, puis homogénéiser (150 rpm) à la même température pendant 1 minute. Laisser refroidir à 40°C et passer le milieu dans un homogénéisateur haute pression (300 bars). Après 1 jour de mûrissement, la viscosité de l'émulsion ainsi formée est de 6160 cp (BROOKFIELD DV II - 25°C - 12 rpm - module 3).

### Exemple 28

### Exemple d'émulsion concentrée phytosanitaire selon l'invention

Chauffer 2 g de l'agent émulsionnant à base de polyglycosides de l'exemple 15, 70 g d'huile de colza et 28 g d'eau osmosée en même temps à une température de 75°C, puis homogénéiser (10000 rpm) à la même température pendant 2 minutes et enfin refroidir sous agitation lente (300 rpm) jusqu'à une température de l'ordre de 25°C. Après 1 jour de mûrissement, la viscosité de l'émulsion ainsi formée est de 1060 cp (BROOKFIELD DV II - 25°C - 12 rpm - module 3). Cette émulsion reste stable pendant 2 mois dans une étuve à 45°C.

### Exemple 29

### Exemple d'émulsion concentrée phytosanitaire selon l'invention

Chauffer 2 g de l'agent émulsionnant à base de polyglycosides de l'exemple 16, 70 g d'ester de colza (ester de Robbe) et 28 g d'eau osmosée en même temps à une température de 75°C, puis homogénéiser (10000 rpm) à la même température pendant 2 minutes et enfin refroidir sous agitation lente (300 rpm) jusqu'à une température de l'ordre de 25°C. Après 1 jour de mûrissement, la viscosité de l'émulsion ainsi formée est de 1800 cp (BROOKFIELD DV II - 25°C - 12 rpm - module 3). Cette émulsion reste stable pendant 2 mois dans une étuve à 45°C.

### Exemple 30

### Exemple d'émulsion diluée phytosanitaire selon l'invention

Dans un tube, on place 0,5 g de l'émulsion de l'exemple 28 et 19,5 g d'eau, puis on mélange le tout par trois retournements successifs du tube. L'émulsion obtenue est stable pendant 2 heures, ce qui correspond au temps nécessaire à l'utilisation du produit par l'agriculteur par exemple.

### Exemple 31

### Exemple d'émulsion de gasoil selon l'invention

Chauffer 1 g de l'agent émulsionnant à base de polyglycosides de l'exemple 15, 69,5 g de gasoil et 29,5 g d'eau osmosée en même temps à une température de 65°C, puis homogénéiser (9500 rpm) à la même température pendant 2 minutes et enfin refroidir sous agitation lente (300 rpm) jusqu'à une température de l'ordre de 25°C.

### Exemple comparatif 32

### Indices de crémage des émulsions préparées à partir des agents émulsionnants à base de polyglycosides et d'alcools gras de l'invention

### Préparation des émulsions

### Compositions :

- 3 % en poids par rapport au poids total de l'émulsion d'agent émulsionnant à base de polyglycosides et d'alcools gras
- 10 % en poids par rapport au poids total de l'émulsion d'huile de tournesol
- 0,5 % en poids par rapport au poids total de l'émulsion de conservateur (Phénonip®)
- eau osmosée qsp 100 % en poids

### Protocole :

Les constituants sont pesés successivement dans un bécher de forme basse et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée à mûrir 24 heures à 25°C avant son analyse.

Tous les agents émulsionnants à base de polyglycosides et d'alcools gras utilisées contiennent 55 % en poids par rapport au poids total des compositions, d'un mélange d'hexadécanol et d'octadécanol (50/50 ; poids/poids). Les polyglycosides sont constitués de polyhexosides (polyglucosides de l'exemple de synthèse 3) et/ou de polypentosides (polyxylosides de l'exemple 17).

### Indice de crémage

L'indice de crémage est défini comme le pourcentage en volume d'émulsion résiduelle après un traitement de déstabilisation par centrifugation. Les centrifugations (4080 g pendant 1 heure) sont identiques pour l'ensemble des essais. Après centrifugation, l'indice de crémage est le rapport du volume d'émulsion résiduelle sur le volume total d'émulsion initiale multiplié par 100.

Les résultats sont résumés dans le tableau suivant :

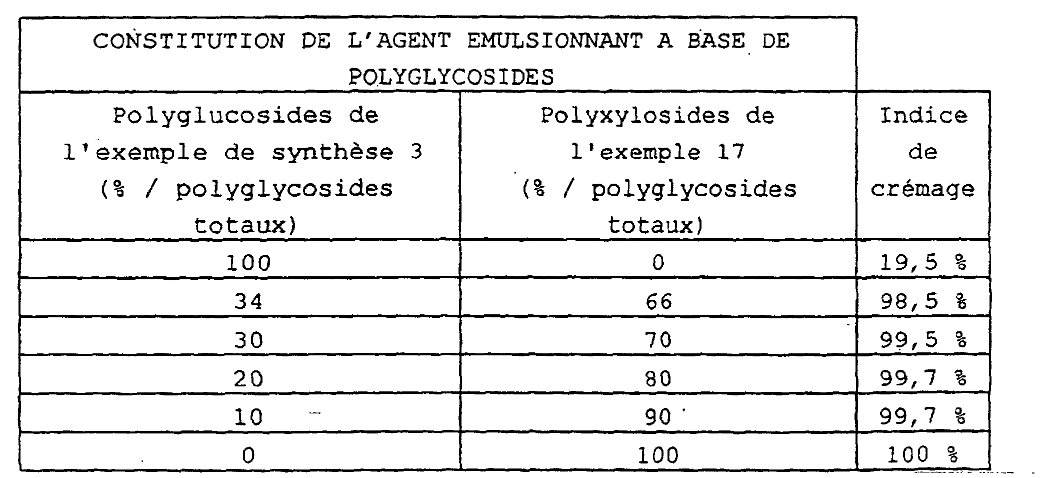

Le tableau précédent permet de mettre en évidence que les agents émulsionnants à base de polyglycosides contenant de 66% à 100% en poids par rapport aux poids total des polyglycosides, de polypentosides et de 0 à 34 % en poids de polyhexosides, permettent de former des émulsions présentant un indice de crémage proche de 100 %, c'est-à-dire stables à la centrifugation ; ce qui n'est pas le cas des compositions contenant exclusivement des polyhexosides tels que les polyglucosides de l'exemple de synthèse 3.

### Exemple 33

### Procédé de préparation d'agent émulsionnant comparatif

Afin de mettre en évidence les propriétés particulières des agents émulsionnants de la présente invention, nous avons préparé l'agent émulsionnant comparatif suivant : 65 g de l'agent émulsionnant à base de polyglucoside de l'exemple 1 de synthèse et 35 g de l'agent émulsionnant à base de polyxylosides de l'exemple 1, sont mélangés afin d'obtenir 100 g d'agent émulsionnant à base de polyglycosides comprenant 35% en poids, par rapport aux polyglycosides, de polypentosides (polyxylosides) et 65 % en poids de polyhexosides (polyglucosides).

### Exemple comparatif 34

### Variation de la rétrodiffusion des émulsions préparées à partir des agents émulsionnants à base de polyglycosides et d'alcools gras de l'invention

### Principe de l'analyse

La méthode d'analyse utilisée pour évaluer les stabilités relatives des émulsions repose sur la mesure de la rétrodiffusion de l'émulsion au moyen d'un TURBISCAN. Cet instrument d'analyse macroscopique à balayage vertical utilise un détecteur proche infrarouge. Il réalise un balayage optique complet de toute la hauteur de l'échantillon d'émulsion et restitue sous forme de graphique ou de tableaux l'aspect macroscopique de l'émulsion à un temps donné. Conçu pour fonctionner en mode cinétique, il détecte les évolutions physiques de l'émulsion par simple comparaison des profils graphiques. Cette technique permet de détecter bien avant l'oeil nu les phénomènes de crémage, de clarification, de sédimentation, de coalescence synonymes de déstabilisation des émulsions. Les émulsions les plus stables sont celles qui présentent une variation de la rétrodiffusion en fonction du temps la plus faible.

### Préparation des émulsions

### Compositions:

- 2 % en poids par rapport au poids total de l'émulsion d'agent émulsionnant
- 30 % en poids par rapport au poids total de l'émulsion d'huile de tournesol
- 0,4 % en poids par rapport au poids total de l'émulsion de conservateur (Phénonip®)
- eau osmosée qsp 100 % en poids.

### Protocole :

Les constituants sont pesés successivement dans un bécher de forme haute et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. Les analyses sont réalisées immédiatement après la fabrication des émulsions.

Tous les agents émulsionnants à base de polyglycosides et d'alcools gras utilisées contiennent 50 % en poids par rapport au poids total des compositions, d'un mélange de tétradécanol et d'octadécanol (25/75 ; poids/poids). Les émulsions comparées sont les suivantes :

| **N° de l'émulsion** | **1** | **2** | **3** | **4** |
|---|---|---|---|---|
| Agent émulsionnant selon l'invention | Exemple 1 | Exemple 11 | - | - |
| Agent émulsionnant comparatif | - | - | Exemple 33 | Exemple de synthèse 1 |
| % de polypentosides / polyglycosides de l'agent émulsionnant | 100% | 90% | 35% | 0% |
| % de polyhexosides / polyglycosides de l'agent émulsionnant | 0% | 10% | 65% | 100% |

Les résultats sont résumés dans le tableau suivant :

### Variation de la rétrodiffusion en fonction du temps mesurée dans le bas des tubes contenant les émulsions à analyser

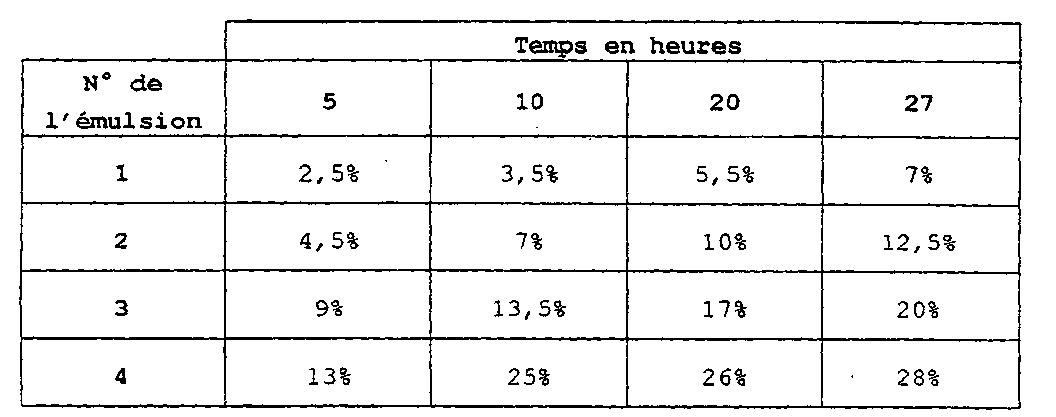

Le tableau précédent permet de montrer que les émulsions préparées à partir des agents émulsionnants de l'invention (N° 1 et 2) présentent des variations de la rétrodiffusion en fonction du temps plus faibles que celles observées avec les émulsions préparées à partir des agents émulsionnants comparatifs (N° 3 et 4). Les émulsions de la présente invention sont donc plus stables.

### 8) Exemple comparatif 35

### Variation de la rétrodiffusion des émulsions préparées à partir des agents émulsionnants à base de polyglycosides et d'alcools gras de l'invention

Cet exemple comparatif est réalisé comme l'exemple comparatif 34 précédent si ce n'est que l'on remplace l'huile de tournesol par le Miglyol 812 N commercialisé par Lambert Rivière.

Les émulsions comparées sont les suivantes :

| **N° de l'émulsion** | **5** | **6** | **7** | **8** |
|---|---|---|---|---|
| Agent émulsionnant selon l'invention | Exemple 1 | Exemple 11 | - | - |
| Agent émulsionnant comparatif | - | - | Exemple 33 | Exemple de synthèse 1 |
| % de polypentosides / polyglycosides de l'agent émulsionnant | 100% | 90% | 35% | 0% |
| % de polyhexosides / polyglycosides de l'agent émulsionnant | 0% | 10% | 65% | 100% |

Les résultats sont résumés dans le tableau suivant :

### Variation de la rétrodiffusion en fonction du temps mesurée dans le bas des tubes contenant les émulsions à analyser

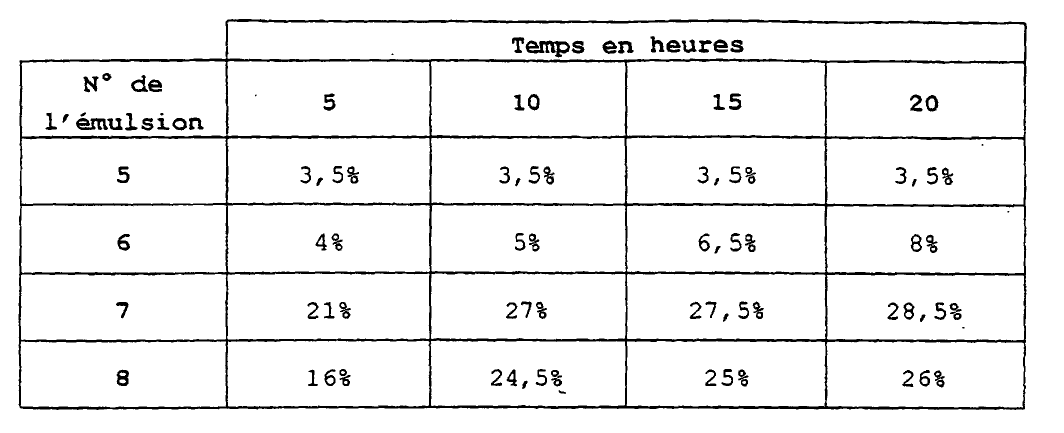

### Pourcentage d'émulsion résiduelle mesuré en fonction du temps par analyse de la rétrodiffusion sur toute la hauteur des tubes contenant les émulsions

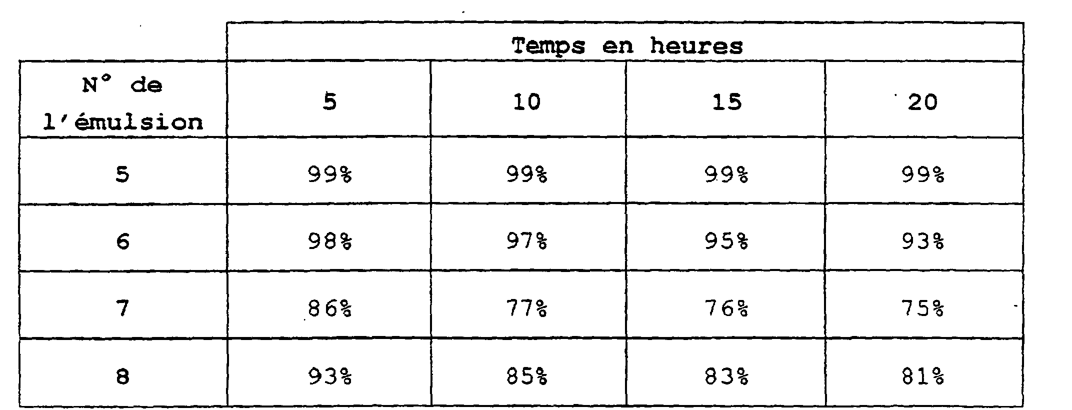

Les tableaux précédents permettent de montrer d'une part, que les émulsions préparées à partir des agents émulsionnants de l'invention (N° 5 et 6) présentent des variations de la rétrodiffusion en fonction du temps plus faibles que celles observées avec les émulsions préparées à partir des agents émulsionnants comparatifs (N° 7 et 8). D'autre part, les émulsions résiduelles obtenues avec les agents émulsionnants de l'invention (N° 5 et 6) sont très largement supérieures à celles obtenues avec les agents comparatifs (N° 7 et 8). Les émulsions de la présente invention sont donc plus stables.

### Exemple comparatif 36

### Indices de crémage des émulsions préparées à partir des agents émulsionnants à base de polyglycosides et d'alcools gras de l'invention

### Préparation des émulsions

### Compositions :

- 2 % en poids par rapport au poids total de l'émulsion d'agent émulsionnant à base de polyglycosides et d'alcools gras
- 30 % en poids par rapport au poids total de l'émulsion d'huile
- 0,4 % en poids par rapport au poids total de l'émulsion de conservateur (Phénonip®)
- eau osmosée qsp 100 % en poids.

### Protocole :

Les constituants sont pesés successivement dans un bécher de forme basse et sont chauffés 10 minutes à 75°C. Le milieu est agité 1 minute à 15 000 rpm au polytron. L'agitation est poursuivie à l'aide d'un barreau magnétique (200 rpm) pendant 30 minutes jusqu'au refroidissement de l'émulsion. L'émulsion est laissée à mûrir 24 heures à 25°C avant son analyse.

Tous les agents émulsionnants à base de polyglycosides et d'alcools gras utilisées contiennent 50 % en poids par rapport au poids total des compositions, d'un mélange de tétradécanol et d'octadécanol (25/75 ; poids/poids).

### Indice de crémage

L'indice de crémage est défini comme le pourcentage en volume d'émulsion résiduelle après un traitement de déstabilisation par centrifugation. Les centrifugations (4080 g pendant 1 heure) sont identiques pour l'ensemble des essais. Après centrifugation, l'indice de crémage est le rapport du volume d'émulsion résiduelle sur le volume total d'émulsion initiale multiplié par 100.

Les émulsions ont été préparées avec deux huiles différentes , le Miglyol 812N commercialiée par Lambert Rivière et l'isostéarate d'isostéaryle de Gattefossé. Les émulsions comparées sont les suivantes :

Avec le Miglyol 812N :

| **N° de l'émulsion** | **9** | **10** | **11** | **12** |
|---|---|---|---|---|
| Agent émulsionnant selon l'invention | Exemple 1 | Exemple 11 | - | - |
| Agent émulsionnant comparatif | - | - | Exemple 33 | Exemple de synthèse 1 |
| % de polypentosides / polyglycosides de l'agent émulsionnant | 100% | 90% | 35% | 0% |
| % de polyhexosides / polyglycosides de l'agent émulsionnant | 0% | 10% | 65% | 100% |

Avec l'isostéarate d'isostéaryle :

| **N° de l'émulsion** | **13** | **14** | **15** | **16** |
|---|---|---|---|---|
| Agent émulsionnant selon l'invention | Exemple 1 | Exemple 11 | - | - |
| Agent émulsionnant comparatif | - | - | Exemple 33 | Exemple de synthèse 1 |
| % de polypentosides / polyglycosides de l'agent émulsionnant | 100% | 90% | 35% | 0% |
| % de polyhexosides / polyglycosides de l'agent émulsionnant | 0% | 10% | 65% | 100% |

Les résultats sont résumés dans le tableau suivant :

| **N° de l'émulsion** | **Indice de crémage (%)** |
|---|---|
| 9 | 100 |
| 10 | 100 |
| 11 | 58 |
| 12 | 58 |
| 13 | 100 |
| 14 | 100 |
| 15 | 66 |
| 16 | 77 |

Le tableau précédent permet de mettre en évidence que les agents émulsionnants de l'invention (N° 9, 10, 13 et 14) permettent de former des émulsions présentant un indice de crémage proche de 100 %, c'est-à-dire stables à la centrifugation ; ce qui n'est pas le cas des émulsions préparees à partir des agents émulsionnants comparatifs (N° 11, 12, 15 et 16).

### Exemple 37

### Mesure de la stabilité à long terme des émulsions de la présente invention par analyse de leur rétrodiffusion

### Principe de l'analyse

Le principe de l'analyse est identique à celui décrit dans l'exemple comparatif 34.

### Préparation des émulsions

### Compositions :

- 4 % en poids par rapport au poids total de l'émulsion d'agent émulsionnant de l'exemple 7
- 30 % en poids par rapport au poids total de l'émulsion d'huile de tournesol
- 0,4 % en poids par rapport au poids total de l'émulsion de conservateur (Phénonip®)
- eau osmosée qsp 100 % en poids.

### Protocole :

Les constituants sont pesés successivement dans un bécher de forme haute et sont chauffés 10 minutes à 75°C. Le milieu est agité 1 minute à 1300 rpm. L'agitation est poursuivie à l'aide d'un barreau magnétique (200 rpm) pendant 30 minutes jusqu'au refroidissement de l'émulsion. Après un jour de mûrissement à 25°C, l'émulsion est placée dans une étuve à 40°C et analysée régulièrement au moyen de l'analyseur de rétrodiffusion (Turbiscan).

Les résultats sont résumés dans le tableau suivant :

| *Pourcentage d'émulsion résiduelle déterminé par analyse de la rétrodiffusion en fonction du temps* | |
|---|---|
| Temps (jours) | % d'émulsion résiduelle |
| 0 | 100 |
| 10 | 100 |
| 30 | 100 |
| 50 | 100 |
| 70 | 100 |

Le tableau précédent permet de montrer que l'émulsion est parfaitement stable dans le temps après un stockage à 40°C.

### Exemple comparatif 38

### Pouvoir suspensif des émulsions préparées à partir des agents émulsionnants à base de polyglycosides et d'alcools gras de l'invention

### Principe

Pour évaluer les pouvoirs suspensifs des émulsions de la présente invention, nous les avons préparées en ajoutant comme additif, des particules solides de tailles comprises entre 0,05 et 0,250 mm. Nous avons ensuite soumis les émulsions à un traitement de déstabilisation par centrifugation à 2000 rpm pendant 3 minutes à 20°C. Nous avons alors mesuré le culot de particules solides relarguées au fond du tube. Les résultats sont exprimés en pourcentage de culot défini par le volume de culot par rapport au volume total d'émulsion centrifugée. Les émulsions qui présentent les meilleurs pouvoirs suspensifs sont celles pour lesquelles nous n'obtenons pas du tout de culot.

### Préparation des émulsions

### Compositions :

- 3 % en poids par rapport au poids total de l'émulsion d'agent émulsionnant à base de polyglycosides et d'alcools gras
- 10 % en poids par rapport au poids total de l'émulsion d'huile (isostéarate d'isostéaryle)
- 5 % en poids de particules solides de 0,05 à 0,25 mm de diamètre
- 0,5 % en poids par rapport au poids total de l'émulsion de conservateur (Phénonip®)
- eau osmosée qsp 100 % en poids.

### Protocole :

Les constituants sont pesés successivement dans un bécher de forme basse et sont chauffés 10 minutes à 75°C. Le milieu est agité 1 minute à 1300 rpm. L'agitation est poursuivie à l'aide d'un barreau magnétique (200 rpm) pendant 30 minutes jusqu'au refroidissement de l'émulsion. L'émulsion est laissée à mûrir 24 heures à 25°C avant son analyse.

Les agents émulsionnants à base de polyglycosides et d'alcools gras utilisées contiennent 50 % en poids par rapport au poids total des compositions, d'un mélange d'hexadécanol et d'octadécanol (33/67 ; poids/poids). Les polyglycosides sont constitués de polyhexosides (polyglucosides de l'exemple de synthèse 2) ou de polypentosides (polyxylosides de l'exemple 2).

Les résultats sont résumés dans le tableau suivant :

| AGENT EMULSIONNANT | POUVOIR SUSPENSIF |
|---|---|
| Agent émulsionnant comparatif de l'exemple de synthèse 2 | 12% |
| Agent émulsionnant de l'exemple 2 de l'invention | 0% |

Le tableau précédent permet de mettre en évidence que l'agent émulsionnant de l'exemple 2 de l'invention permet de former des émulsions présentant un pouvoir suspensif excellent, c'est-à-dire absence de culot obtenu après centrifugation ; ce qui n'est pas le cas de l'agent émulsionnant comparatif de l'exemple de synthèse 2 pour lequel les émulsions relarguent un culot de 12%.

### Exemple 39

### Crème auto-bronzante et hydratante résistante à l'eau

| | | |
|---|---|---|
| A - | Agent émulsionnant de l'exemple 4 | 4,0 % |
| | Aloe vera | 1,0 % |
| | Beurre de karité | 0,2 % |
| | Diméthicone | 2,0 % |
| | 2-octyl dodécyl myristate (MOD) | 3,0 % |
| | Propylglycol stéarate (Stepan PGMS) | 1,0 % |
| | Acide stéarique | 1,0 % |
| | Vitamine E | 0,1 % |
| | Acide hyaluronique (VITALHYAL) | 1,0 % |
| | Phénonip | 0,5 % |
| B - | Glycérol | 10 % |
| | Eau | qsp 100 % |
| C - | Dihydroxyacétone | 5,0 % |
| | Eau | 10,0% |
| D - | Fragrance | QS |

Procédé de fabrication de la crème :
Peser tous les ingrédients de A
Peser tous les ingrédients de B et homogénéiser
Chauffer à 75 °C séparément
Mettre A sous agitation à 800 rpm
Ajouter B en filet dans A
Mélanger à 1300 rpm quelques minutes à 75 °C
Laisser refroidir à 40°C en agitant à 300 rpm
Préparer la solution C à température ambiante
Additionner C et D dans l'émulsion
Corriger le pH si cela est nécessaire.

### Exemple 40

### Lait hydratant

| | |
|---|---|
| Agent émulsionnant de l'exemple 10 | 2,0 % |
| Miglyol 812 N | 3,0 % |
| Isostéarate d'isostéaryle | 3,0 % |
| Diméthicone | 2,0 % |
| Acide stéarique | 1,0 % |
| Acide hyaluronique (VITALHYAL) | 1,0 % |
| Phénonip | 0,5 % |
| Eau | QSP 100 % |

### Procédé de fabrication du lait :

Peser tous les ingrédients
Chauffer à 75 °C
Mélanger à 3000 rpm quelques minutes à 75 °C
Laisser refroidir à 30°C en agitant à 500 rpm
Corriger le pH.si cela est nécessaire.

### Exemple 41

### Lait démaquillant

| | | |
|---|---|---|
| A - | Agent émulsionnant de l'exemple 7 | 4,0 % |
| | Beurre de karité | 2,0 % |
| | Diméthicone | 2,0 % |
| | Huile d'amande douce | 2,0 % |
| | Huile de Jojoba | 3,0 % |
| | Huile de coton hydrogénée | 3,0 % |
| B - | Vitamine E | 0,5 % |
| | Vitalhyal | 5,0% |
| | Muciliance | 0,05% |
| | Peptides de blé | 0,5% |
| | Solavena | 1,0% |
| | Phénonip | 0,4% |
| | Eau | qsp 100 % |
| C - | Gelwhite (3% dans l'eau) | 10,0 % |
| D - | Parfum | QS |
| | Colorant | QS |

### Procédé de fabrication du lait :

Peser tous les ingrédients de A et chauffer à 75°C
Peser tous les ingrédients de B et chauffer à 75°C sous agitation
Ajouter B dans A sous agitation à 800 rpm et à 75°C
Agiter 2 minutes à 1300 rpm
Agiter à 200 rpm jusqu'à 40°C
Ajouter C
Corriger le pH si cela est nécessaire
Ajouter D.

### Exemple 42

### Crème de nuit

| | |
|---|---|
| Agent émulsionnant de l'exemple 8 | 4,0 % |
| Diméthicone | 2,0 % |
| Huile de jojoba | 1,5 % |
| Huile d'amandes douces | 1,5 % |
| MOD | 5,0 % |
| Acide stéarique | 2,0 % |
| Huile de blé | 1,0 % |
| Solavena | 1,0 % |
| Vitalhyal (acide hyaluronique) | 2,0 % |
| Vitamine E | 0,5 % |
| Phénonip | 0,5 % |
| Fragrance | QS |
| Colorant | QS |
| Eau | QSP 100 % |

### Procédé de fabrication de la crème :

Peser tous les ingrédients excepté la fragrance
Chauffer à 75 °C
Mélanger à 300 rpm quelques minutes jusqu'à 40 °C
Ajuster le pH
Additionner la fragrance
Passer dans un homogénéisateur à 300 bars.

### Exemple 43

### Crème fluide gommante

| | |
|---|---|
| Agent émulsionnant de l'exemple 9 | 4,0 % |
| Miglyol 812 N | 10,0 % |
| Acide hyaluronique (Vitalhyal) | 2,0 % |
| Exfoliance | 5,0 % |
| Phénonip | 0,5 % |
| Fragrance | QS |
| Eau | QSP 100 % |

### Procédé de fabrication de la crème fluide :

Peser tous les ingrédients excepté la fragrance
Chauffer à 50 °C
Mélanger à 1300 rpm quelques minutes
Laisser refroidir en agitant à 300 rpm
A 25°C, additionner la fragrance
Corriger le pH si cela est nécessaire.

### Exemple 44

### Crème gel contour des yeux

| | | |
|---|---|---|
| A. | Acide mucique (Muciliance) | 0,05 % |
| | Eau | QSP 100 % |
| | | |
| B. | Agent émulsionnant de l'exemple 12 | 3,0 % |
| | Bashyal | 1, 0 % |
| | Vitalhyal | 2,0 % |
| | Phénonip | 0,4 % |
| | | |
| c. | Eau de bleuet | 10,0 % |

### Procédé

- Mélanger les ingrédients de A
- Ajuster le pH à 6 avec de la soude 1N
- Ajouter B dans A
- Chauffer à 75°C
- Agiter 1 minute à 500 rpm.
- Laisser refroidir sous agitation à 300 rpm
- Ajouter C à 30°C.

### Exemple 45

### Baume nutritif pour cheveux

| | |
|---|---|
| Agent émulsionnant de l'exemple 14 | 3,0 % |
| Diméthicone | 1,0 % |
| Huile de blé | 0,5 % |
| Peptides de blé | 0,5 % |
| Phénonip | 0,5 % |
| Parfum | QS |
| Eau | QSP 100 % |

### Procédé de fabrication :

- Peser tout sauf le parfum
- Chauffer 75°C
- Agiter à 1300 rpm 1 minute
- Laisser refroidir à 300 rpm jusqu'à 25°C
- Ajouter le parfum.

### Exemple 46

### Crème anti-acnée

| | |
|---|---|
| Composition de l'exemple 13 | 4,0 % |
| Huile de paraffine (MARCOL 82) | 2,0 % |
| Miglyol 812 N | 3,0 % |
| Isostéarate d'isostéaryle | 3,0 % |
| Diméthicone | 2,0 % |
| Acide stéarique | 2,0 % |
| Sophoroses lipides (SOPHOLIANCE) | 1,0 % |
| Phénonip | 0,5 % |
| Eau | QSP 100 % |

### Procédé de fabrication de la crème :

Préparer une solution aqueuse limpide de SOPHOLIANCE à 25 % de matière sèche, à pH 6 (NaOH).
Peser tous les ingrédients excepté SOPHOLIANCE
Chauffer à 75 °C pendant 10 minutes
Mélanger à 1500 rpm 1 minute à 75 °C
Laisser refroidir en agitant à 300 rpm
Additionner SOPHOLIANCE vers 50°C
Corriger le pH si cela est nécessaire.
Stopper l'agitation vers 30°C

## Revendications

1. Emulsion comprenant, en poids :
- de 4,5 à 99,5% d'eau
- de 0 à 95% d'huile
- de 0 à 50% de substance active
- un agent émulsionnant en complément à 100%,
l'agent émulsionnant comprend de 30 à 65% en poids d'au moins un alcool gras de formule ROH, où R est un radical aliphatique, saturé ou insaturé ayant de 1 à 4 insaturations éthyléniques, linéaire ou ramifié, ayant de 12 à 22 atomes de carbone, le reste étant, sauf des impuretés, constitué :
a)- soit, d'un mélange de polyglycosides comprenant des polypentosides de formule (I) :
R¹O(P)ₙ₁ (I)
dans laquelle R¹ est un radical aliphatique, saturé ou insaturé ayant de 1 à 4 insaturations éthyléniques, linéaire ou ramifié, ayant de 12 à 22 atomes de carbone, p est le reste d'un pentose choisi parmi l'arabinose et le xylose, n1 est compris entre 1 et 5 ; et des polyhexosides de formule (II) :
R²O(H)ₙ₂ (II)
dans laquelle R² est un radical aliphatique, saturé ou insaturé ayant de 1 à 4 insaturations éthyléniques, linéaire ou ramifié, ayant de 12 à 22 atomes de carbone, H est le reste d'un hexose, n2 est compris entre 1 et 5 ;
b)- soit d'un mélange de polyglycosides de formule (III):
R³O(G₁)ₐ(G₂)_{b}(G₃)_{c}(G₄)_{d}(G₅)ₑ (III)
dans laquelle R³ est un radical aliphatique, saturé ou insaturé ayant de 1 à 4 insaturations éthyléniques, linéaire ou ramifié, ayant de 12 à 22 atomes de carbone, G₁, G₂, G₃, G₄, G₅ sont indépendamment les uns des autres, des restes d'un ose choisi parmi les hexoses et les pentoses, ces derniers étant choisis parmi l'arabinose et le xylose ; a, b, c, d et e étant égaux à 0 ou à 1, la somme de a, b, c, d et e étant au moins égale à 1
c)- soit d'un mélange de a et b
**caractérisée par** les deux caractéristiques 1 et 2 suivantes :
1)lorsque l'agent émulsionnant comprend des polyglycosides comprenant des polypentosides de formule (I) et des polyhexosides de formule (II), les polypentosides de formule (I) représentent 66 à 100% en poids de l'ensemble des polyglycosides, et les polyhexosides de formule (II) 0 à 34% en poids de l'ensemble des polyglycosides, lorsque l'agent émulsionnant comprend des polyglycosides de formule (III), les pentoses représentent alors 66 à 100% en poids par rapport à l'ensemble des oses G₁, G₂, G₃, G₄ et G₅ et les hexoses de 0 à 34% en poids par rapport à l'ensemble des oses G₁, G₂, G₃, G₄ et G₅ et
2-a) ou bien l'agent émulsionnant représente au moins 2,5 % du poids total de l'émulsion
2-b) ou bien l'agent émulsionnant représente de 1 à 2,5 % du poids total de l'émulsion et l'huile est choisie parmi :
- l'huile d'amandes douces, l'huile de coprah, l'huile de ricin, l'huile de jojoba, l'huile d'olive, l'huile de colza, l'huile d'arachide, l'huile de noisette, l'huile de palme, l'huile de noyau d'abricot, l'huile de calophyllum, l'huile de carthame, l'huile d'avocat, l'huile de pépins de cassis, l'huile de tournesol, l'huile de germes de maïs, l'huile de soja, l'huile de coton, l'huile de luzerne, l'huile d'orge, l'huile de pépins de raisin, l'huile de pavot, l'huile de potimarron, l'huile de sézame, l'huile de seigle, l'huile d'onagre, l'huile de passiflore, des dérivés de ces huiles, les huiles hydrogénées,
- les huiles d'origine animale,
- les huiles synthétiques, les poly-α-oléfines,
- les dérivés de la lanoline,
- les alcanediols possédant de 2 à 10 atomes de carbone,
- les alcools de formule R⁴-OH où R⁴ est un radical aliphatique, saturé ou insaturé ayant de 1 à 4 insaturations éthyléniques, linéaire ou ramifié, ayant de 12 à 22 atomes de carbone,
- les polyéthylène glycols, les polypropylène glycols,
- les esters gras de formule R⁵-0-CO- R⁶ où R⁵ et R⁶ sont indépendamment l'un de l'autre des radicaux aliphatiques, saturés ou insaturés ayant de 1 à 4 insaturations éthyléniques, linéaires ou ramifiés, ayant de 1 à 22 atomes de carbone,
- et les huiles silicones.

2. Emulsion selon la revendication 1, **caractérisée en ce que** l'agent émulsionnant est le seul en tant qu'agent émulsionnant.

3. Emulsion selon la revendication 1 ou 2, **caractérisée en ce que** l'agent émulsionnant représente de 2,5 à 10% et de préférence de 2,5 à 6% et plus particulièrement de 2,5 à 4% du poids total de l'émulsion.

4. Emulsion selon la revendication 1,2 ou 3, **caractérisée en ce que** l'agent émulsionnant lorsqu'il comprend des polyglycosides comprenant des polypentosides de formule (I) et des polyhexosides de formule (II), les polypentosides de formule (I) représentent 80 à 100% et de préférence 97 à 100% en poids de l'ensemble des polyglycosides, et les polyhexosides de formule (II) 0 à 20% et de préférence 0 à 3% en poids de l'ensemble des polyglycosides et lorsqu'il comprend des polyglycosides de formule (III), les pentoses représentent alors 80 à 100% et de préférence 97 à 100% en poids par rapport à l'ensemble des oses G₁, G₂, G₃, G₄ et G₅ et les hexoses de 0 à 20% et de préférence 0 à 3% en poids par rapport à l'ensemble des oses G₁, G₂, G₃, G₄ et G₅.

5. Emulsion selon la revendication 1, 2, 3 ou 4, **caractérisée en ce que** les polypentosides ou les restes de pentoses sont constitués respectivement par au moins 85 % de polyxylosides ou de restes de xylose.

6. Emulsion selon l'une des revendications 1 à 5, **caractérisée en ce que** les polypentosides ou les restes de pentoses sont respectivement des polyxylosides et du xylose.

7. Emulsion selon l'une des revendications 1 à 6, **caractérisée en ce que** les polyhexosides ou les restes d'hexoses sont constitués respectivement par au moins 80 % de polyglucosides ou de restes de glucose.

8. Emulsion selon l'une des revendications 1 à 7, **caractérisée en ce que** l'agent émulsionnant comprend des polyglycosides de formules (I), (II) ou (III) dont les radicaux R¹ et R² ou le radical R³ sont identiques au radical R de l'alcool gras.

9. Emulsion selon l'une des revendications 1 à 8, **caractérisée en ce que** l'alcool gras de l'agent émulsionnant présente de 14 à 22 atomes de carbone et consiste de préférence en un mélange d'alcools ayant de 14 à 18 atomes de carbone.

10. Emulsion selon l'une des revendications 1 à 9, caractérisée en que l'agent émulsionnant comprend de 40 à 60 % d'alcool gras et de préférence de 47 à 52 % d'alcool gras.

11. Emulsion selon l'une des revendications 1 à 10, **caractérisée par le fait que** la phase huileuse représente de 2 à 60 % et de préférence 10 à 30 % en poids par rapport au poids total de l'émulsion.

12. L'utilisation d'une émulsion suivant l'une des revendications 1 à 11 dans une composition cosmétique ou pharmaceutique.

13. L'utilisation d'une émulsion suivant l'une des revendications 1 à 11 dans une composition phytosanitaire.

14. Agent émulsionnant **caractérisé en ce qu'**il comprend des polyglycosides de formule (III),
R³O(G₁)ₐ(G₂)_{b}(G₃)_{C}(G₄)_{d}(G₅)ₑ (III)
dans laquelle R³ est un radical aliphatique, saturé ou insaturé ayant de 1 à 4 insaturations éthyléniques, linéaire ou ramifié, ayant de 12 à 22 atomes de carbone, G₁, G₂, G₃, G₄, G₅ sont indépendamment les uns des autres, des restes d'un ose choisi parmi les hexoses et les pentoses, ces derniers étant choisis parmi l'arabinose et le xylose ; a, b, c, d et e étant égaux à 0 ou à 1, la somme de a, b, c, d et e étant au moins égale à 1, les pentoses représentant alors 97 à 100% en poids par rapport à l'ensemble des oses G₁, G₂, G₃, G₄ et G₅ et les hexoses de 0 à 3% en poids par rapport à l'ensemble des oses G₁, G₂, G₃, G₄ et G₅, le D-xylose représentant au moins 95% en poids de l'ensemble des pentoses, mais ne représentant pas 100% des oses G₁, G₂ G₃, G₄ et G₅.

## Patentansprüche

1. Emulsion aufweisend in Gew.%:
- 4,5 bis 99,5 % Wasser
- 0 bis 95% Öl
- 0 bis 50% Wirksubstanz
- einen Emulgator ad 100%,
wobei der Emulgator mindestens 30 bis 65 Gew.% eines Fettalkohols der Formel ROH aufweist, R ein aliphatischer Rest ist, gesättigt oder ungesättigt mit 1 bis 4 ethylenischen Unsättigungen, gerad- oder verzweigtkettig, mit 12 bis 22 Kohlenstoffatomen, wobei der Rest außer Verunreinigungen sich zusammensetzt aus:
a) - entweder einer Mischung von Polyglycosiden, aufweisend Polypentoside der Formel (I):
R¹-O(P)ₙ₁ (I)
wobei R¹ ein aliphatischer Rest ist, gesättigt oder ungesättigt mit 1 bis 4 ethylenischen Unsättigungen, gerad- oder verzweigtkettig mit 12 bis 22 Kohlenstoffatomen, P ein Pentoserest ist, ausgewählt aus Arabinose und Xylose, mit n1 von 1 bis 5; und Polyhexosiden der Formel (II)
R²-O(H)ₙ₂ (II)
wobei R² ein aliphatischer Rest ist, gesättigt oder ungesättigt mit 1 bis 4 ethylenischen Unsättigungen, gerad- oder verzweigtkettig mit 12 bis 22 Kohlenstoffatomen, H ein Hexoserest, mit n2 von 1 bis 5;
b) - oder aus einer Mischung von Polyglycosiden der Formel (III)
R³O(G₁)ₐ(G₂)_{b}(G₃)_{c}(G₄)_{d}(G₅)ₑ (III)
wobei R³ ein aliphatischer Rest ist, gesättigt oder ungesättigt mit 1 bis 4 ethylenischen Unsättigungen, gerad- oder verzweigtkettig, mit 12 bis 22 Kohlenstoffatomen, worin G₁, G₂, G₃, G₄, G₅ unabhängig voneinander Rest einer -ose sind, ausgewählt aus Hexosen und Pentosen,
letzere ausgewählt aus Arabinose und Xylose; wobei a, b, c, d und e gleich 0 oder 1 sind, wobei die Summe von a, b, c, d und e mindestens gleich 1 ist.
c) - oder aus einer Mischung von a) und b) **gekennzeichnet durch** die folgenden Merkmale 1 und 2:
1) wenn der Emulgator Polypentoside der Formel (I) und Polyhexoside der Formel (II) als Polyglycoside aufweist, repräsentieren die Polypentoside der Formel (I) 66 bis 100 Gew.% der gesamten Polyglycoside und die Polyhexoside der Formel (II) 0 bis 34 Gew.% der gesamten Polyglycoside, wenn der Emulgator Polyglycoside der Formel (III) aufweist, repräsentieren die Pentosen dann 66 bis 100 Gew.% bezogen auf die Gesamtheit der -osen G₁, G₂, G₃, G₄, G₅ und die Hexosen 0 bis 34 Gew.% bezogen auf die Gesamtheit der-osen G₁, G₂, G₃, G₄, G und
2a) entweder repräsentiert der Emulgator rmindestens 2,5 Gew.% der ganzen Emulsion
2b) oder repräsentiert der Emulgator 1 bis 2,5 Gew.% der ganzen Emulsion und das Öl wird ausgewählt aus:
- Süßmandelöl, Coprah-Öl, Rizinusöl, Jojobaöl, Olivenöl, Rapsöl, Emußöl, Nußöl, Palmöl, Aprikosenkemöl, Calophyllumöl, Färberdistelöt, Avocadoöl, Kemöl der Schwarzen Johannisbeere, Sonnenblumenöl, Maiskeimöl, Sojaöl, Baumwollöl, Luzemenöl, Gerstenöl, Traubenkemöl, Mohnöl, Potimarronkürbisöl, Sesamöl, Roggenöl, Nachtkerzenöl, Passionsblumenöl, Derivate dieser Öle, hydrogenierte Öle,
- Öle tierischen Ursprungs
- Synthetische Öle, Poly-α-Olefine
- Derivate von Lanolin
- Alkandiole mit 2 bis 10 Kohlenstoffatomen
- Alkohole der Formel R⁴-OH, wobei R⁴ ein aliphatischer Rest ist, gesättigt oder ungesättigt mit 1 bis 4 ethylenischen Unsättigungen, gerad- oder verzweigtkettig mit 12 bis 22 Kohlenstoffatomen, Polyethylenglykole, Polypropylenglykole
- Fettsäureester der Formel R⁵-O-CO- R⁶, wobei R⁵ und R⁶ unabhängig voneinander aliphatische Reste sind, gesättigt oder ungesättigt mit 1 bis 4 ethylenischen Unsättigungen, gerad- oder verzweigtkettig mit 12 bis 22 Kohlenstoffatomen,
- Silikonöle.

2. Emulsion nach Anspruch 1, **dadurch gekennzeichnet, dass** der Emulgator der einzige Emulgator ist.

3. Emulsion nach Anspruch 1 oder 2, **dadurch gekennzeichnet, dass** der Emulgator 2,5 bis 10 Gew.% , bevorzugt 2,5 bis 6 Gew.% und besonders bevorzugt 2,5 bis 4 Gew.%, bezogen auf die gesamte Emulsion, darstellt.

4. Emulsion nach Anspruch 1, 2 oder 3 **dadurch gekennzeichnet, dass**, falls der Emulgator aufweist:
Polypentoside der Formel (I) und Polyhexoside der Formel (II) als Polyglycoside, die Polypentoside der Formel (I) 80 bis 100 Gew.%, bevorzugt 97 bis 100 Gew.% der gesamten Polyglycoside repräsentieren und die Polyhexoside der Formel (II) 0 bis 20 Gew.%, bevorzugt 0 bis 3 Gew.% der gesamten Polyglycoside, oder
Polyglycoside der Formel (III), die Pentosen dann 80 bis 100 Gew.%, bevorzugt 97 bis 100 Gew% bezogen auf die Gesamtheit der -osen G₁, G₂, G₃, G₄, G₅ und die Hexosen 0 bis 20 Gew.%, bevorzugt 0 bis 3 Gew.% bezogen auf die Gesamtheit der -osen G₁, G₂, G₃, G₄, G₅ repräsentieren.

5. Emulsion nach Anspruch 1, 2, 3 oder 4 . **dadurch gekennzeichnet, dass** die Polypentoside oder die Pentosereste zu mindestens 85% aus Polyxylosiden beziehungsweise Xyloseresten bestehen.

6. Emulsion nach einem der Ansprüche 1 bis 5 , **dadurch gekennzeichnet, dass** die Polypentoside oder die Pentosereste Polyxyloside beziehungsweise Xylosereste sind.

7. Emulsion nach einem der Ansprüche 1 bis 6 , **dadurch gekennzeichnet, dass** die Polyhexoside oder die Hexosereste zu mindestens aus 80% aus Polyglycosiden beziehungsweise Glucoseresten bestehen.

8. Emulsion nach einem der Ansprüche 1 bis 7, **dadurch gekennzeichnet, dass** der Emulgator Polyglycoside der Formeln (I), (II) oder (III) aufweist, wobei die Reste R¹ und R² oder der Rest R³ identisch mit dem Rest R des Fettalkohols sind.

9. Emulsion nach einem der Ansprüche 1 bis 8, **dadurch gekennzeichnet, dass** der Fettalkohol des Emulgators14 bis 22 Kohlenstoffatome aufweist und sich bevorzugt aus einer Mischung von Alkoholen mit 14 bis 18 Kohlenstoffatomen zusammensetzt.

10. Emulsion nach einem der Ansprüche 1 bis 9, **dadurch gekennzeichnet, dass** der Emulgator 40 bis 60% Fettalkohol und bevorzugt 47 bis 52 % Fettalkohol aufweist.

11. Emulsion nach einem der Ansprüche 1 bis 10, **gekennzeichnet durch** die Tatsache, dass die Ölphase 2 bis 60% und bevorzugt 10 bis 30 Gew.% bezogen auf das Gesamtgewicht der Emulsion darstellt.

12. Verwendung einer Emulsion nach einem der Ansprüche 1 bis 11 in einer kosmetischen oder pharmazeutischen Formulierung.

13. Verwendung einer Emulsion nach einem der Ansprüche 1 bis 11 in einer Pflanzenschutzformulierung.

14. Emulgator **dadurch gekennzeichnet, daß** er Polyglycoside der Formel (III) aufweist,
R³O(G₁)ₐ(G₂)_{b}(G₃)_{c}(G₄)_{d}(G₅)ₑ (III)
wobei R³ ein aliphatischer Rest ist, gesättigt oder ungesättigt mit 1 bis 4 ethylenischen Unsättigungen, gerad- oder verzweigtkettig, mit 12 bis 22 Kohlenstoffatomen; G₁, G₂, G₃, G₄, G₅ unabhängig voneinander Reste einer - ose , ausgewählt aus Hexosen und Pentosen, letzere ausgewählt aus Arabinose und Xylose, sind; wobei a, b, c, d und e gleich 0 oder 1 sind, wobei die Summe von a, b, c, d und e mindestens gleich 1 ist; die Pentosen daher 97% bis 100 Gew.% bezogen auf die Gesamtheit der -osen G₁, G₂, G₃, G₄, und G₅ und die Hexosen 0 bis 3 Gew.% bezogen auf die Gesamtheit der -osen G₁, G₂, G₃, G₄, und G₅ repräsentieren; wobei D-Xylose mindestens 95 Gew.% bezogen auf die Gesamtheit der Pentosen darstellt, aber nicht 100% der -osen G₁, G₂, G₃, G₄, und G₅ darstellt.

## Claims

1. Emulsion containing, by weight:
- 4.5 to 99.5% of water
- 0 to 95% of oil
- 0 to 50% of active substance
- an emulsifier making up the total of 100%,
the emulsifier contains 30 to 65% by weight of at least one fatty alcohol of formula ROH, where R is a straight-chained or branched aliphatic radical, which may be saturated or unsaturated, having 1 to 4 ethylenic unsaturations, having 12 to 22 carbon atoms, the remainder consisting, except for impurities, of:
a) either a mixture of polyglycosides comprising polypentosides of formula (I):
R¹O(P)ₙ₁ (I)
wherein R¹ is a straight-chained or branched, saturated or unsaturated aliphatic radical having 1 to 4 ethylenic unsaturations, with 12 to 22 carbon atoms, P is the residue of a pentose selected from arabinose and xylose, n1 is between 1 and 5; and polyhexosides of formula (II) :
R²O(H)ₙ₂ (II)
wherein R² is a straight-chained or branched, saturated or unsaturated aliphatic radical having 1 to 4 ethylenic unsaturations, with 12 to 22 carbon atoms, H is the residue of a hexose, n2 is between 1 and 5;
b) or a mixture of polyglycosides of formula (III):
R³O(G₁)ₐ(G₃)_{b}(G₃)_{c}(G₄)_{d}(G₅)ₑ (III)
wherein R³ is a straight-chained or branched, saturated or unsaturated aliphatic radical having 1 to 4 ethylenic unsaturations and 12 to 22 carbon atoms, G₁, G₂, G₃, G₄ and G₅ independently of one another are residues of an ose selected from among the hexoses and pentoses, the latter being selected from arabinose and xylose; a, b, c, d and e being equal to 0 or 1, the sum of a, b, c, d and e being at least 1
c) or a mixture of a and b
**characterised by** the following two features 1 and 2:
1) when the emulsifier contains polyglycosides comprising polypentosides of formula (I) and polyhexosides of formula (II), the polypentosides of formula (I) represent 66 to 100% by weight of the total polyglycosides, and the polyhexosides of formula (II) constitute 0 to 34% by weight of the total polyglycosides, when the emulsifier comprises polyglycosides of formula (III), the pentoses then constitute 66 to 100% by weight based on the total oses G₁, G₂, G₃, G₄ and G₅ and the hexoses constitute from 0 to 34% by weight based on the total oses G₁, G₂, G₃, G₄ and G₅ and
2-a) either the emulsifier constitutes at least 2.5% of total weight of the emulsion
2-b) or the emulsifier constitutes from 1 to 2.5% of the total weight of the emulsion and the oil is selected from among:
- sweet almond oil, coconut oil, castor oil, jojoba oil, olive oil, rape seed oil, groundnut oil, hazelnut oil, palm oil, apricot kernel oil, calophyllum oil, safflower oil, avocado oil, blackcurrant seed oil, sunflower oil, corn seed oil, soya oil, cotton seed oil, alfalfa oil, barley oil, grapeseed oil, poppy oil, pumpkin oil, sesame oil, rye oil, evening primrose oil, passionflower oil, derivatives of these oils, hydrogenated oils,
- oils of animal origin,
- synthetic oils, poly-α-olefines,
- lanolin derivatives,
- alkanediols having 2 to 10 carbon atoms,
- alcohols of formula R⁴-OH where R⁴ is a straight-chained or branched, saturated or unsaturated aliphatic radical having 1 to 4 ethylenic unsaturations and 12 to 22 carbon atoms,
- polyethylene glycols, polypropylene glycols,
- the fatty esters of formula R⁵-O-CO- R⁶ wherein R⁵ and R⁶ independently of each other denote straight-chained or branched, saturated or unsaturated aliphatic radicals having 1 to 4 ethylenic unsaturations, with 1 to 22 carbon atoms,
- and the silicone oils.

2. Emulsion according to claim 1, **characterised in that** the emulsifier is the only emulsifier present.

3. Emulsion according to claim 1 or 2, **characterised in that** the emulsifier represents 2.5 to 10% and preferably 2.5 to 6%, more particularly 2.5 to 4% of the total weight of the emulsion.

4. Emulsion according to claim 1, 2 or 3,
**characterised in that** when the emulsifier contains polyglycosides comprising polypentosides of formula (I) and polyhexosides of formula (II), the polypentosides of formula (I) represent 80 to 100% and preferably 97 to 100% by weight of the total polyglycosides, and the polyhexosides of formula (II) represent 0 to 20% and preferably 0 to 3% by weight of the total polyglycosides and when the emulsifier contains polyglycosides of formula (III) the pentoses represent 80 to 100% and preferably 97 to 100% by weight, based on the total oses G₁, G₂, G₃, G₄ and G₅ and the hexoses represent 0 to 20% and preferably 0 to 3% by weight based on the total oses G₁, G₂, G₃, G₄ and G₅.

5. Emulsion according to claim 1, 2, 3 or 4,
**characterised in that** the polypentosides or the pentose groups consist, respectively, of at least 85% of polyxylosides or xylose groups.

6. Emulsion according to one of claims 1 to 5,
**characterised in that** the polypentosides or the pentose groups are polyxylosides and xylose, respectively.

7. Emulsion according to one of claims 1 to 6,
**characterised in that** the polyhexosides or the hexose groups consist respectively of at least 80% of polyglucosides or glucose groups.

8. Emulsion according to one of claims 1 to 7,
**characterised in that** the emulsifier contains polyglycosides of formulae (I), (II) or (III) wherein the radicals R¹ and R² or the radical R³ are identical to the radical R of the fatty alcohol.

9. Emulsion according to one of claims 1 to 8,
**characterised in that** the fatty alcohol of the emulsifier has 14 to 22 carbon atoms and preferably consists of a mixture of alcohols having 14 to 18 carbon atoms.

10. Emulsion according to one of claims 1 to 9,
**characterised in that** the emulsifying agent contains 40 to 60% of fatty alcohol and preferably 47 to 52% of fatty alcohol.

11. Emulsion according to one of claims 1 to 10,
**characterised in that** the oily phase represents 2 to 60% and preferably 10 to 30% by weight, based on the total weight of the emulsion.

12. Use of an emulsion according to one of claims 1 to 11 in a cosmetic or pharmaceutical composition.

13. Use of an emulsion according to one of claims 1 to 11 in a plant-protection composition.

14. Emulsifier, **characterised in that** it contains polyglycosides of formula (III),
R³O(G₁)ₐ(G₂)_{b}(G₃)_{c}(G₄)_{d}(G₅)ₑ (III)
wherein R³ is a straight-chained or branched, saturated or unsaturated aliphatic radical having 1 to 4 ethylenic unsaturations and 12 to 22 carbon atoms, G₁, G₂, G₃, G₄ and G₅ independently of one another are residues of an ose selected from among the hexoses and pentoses, the latter being selected from arabinose and xylose; a, b, c, d and e being equal to 0 or 1, the sum of a, b, c, d and e being at least 1, the pentoses representing 97 to 100% by weight, based on the total oses G₁, G₂, G₃, G₄ and G₅ and the hexoses representing 0 to 3% by weight, based on the total oses G₁, G₂, G₃, G₄ and G₅, D-xylose representing at least 95% by weight of the total pentoses, but not representing 100% of the oses G₁, G₂, G₃, G₄ and G₅.
